# EUROPEAN PATENT SPECIFICATION

(11) **EP 3 332 343 B1**
(45) Date of publication and mention of the grant of the patent: **20.10.2021**
(21) Application number: 16754034.3
(22) Date of filing: 02.08.2016
(51) Int. Cl.: G16H 20/10, G16H 70/40

(54) **AUTOMATIC PRESCRIPTION MEDICATION SCHEDULING**
AUTOMATISCHE ZEITPLANUNG VON REZEPTPFLICHTIGEN MEDIKAMENTEN
PLANIFICATION AUTOMATIQUE DE MÉDICAMENT SUR ORDONNANCE

(30) Priority: 03.08.2015 US 201514816834
(43) Date of publication of application: 13.06.2018
(73) Proprietor: CVS Pharmacy, Inc., Woonsocket, RI 02895 (US)
(72) Inventor: NICHOLS, Linda, Long Valley, NJ 07981 (US); ACHARYA, Reena, Boston, MA 02111 (US); LIZOTTE, Margaret M., Holden, MA 01520 (US); ST. JEAN, Donna L., North Grafton, MA 01536 (US); LOVERING, Kimberly A., Glen Ellyn, IL 60137 (US)
(74) Representative: Anderson, Oliver Ben
(86) International application number: PCT/US2016/045142
(87) International publication number: WO 2017/023919

(56) References cited:
- US-A1- 2011 000 170
- US-A1- 2012 016 687
- Anastasios Alexiadis: "Scheduling and Planning algorithms for Electronic Calendar Management", , 1 January 2013 (2013-01-01), XP055604985, Retrieved from the Internet: URL:https://pdfs.semanticscholar.org/40ba/ 94d700f732064bb8c638ed00ed892ee8fedb.pdf [retrieved on 2019-07-12]

## Description

### Field of the Disclosure

The disclosure related generally to the field of health care and, more particularly, to a system and method for automatically scheduling medication regimens for patients.

### Background of the Disclosure

Patients needing multiple simultaneous medications often have complicated schedules for when to take each medication. For example, a patient may have a first medication that needs to be taken once daily, a second medication that needs to be taken two times daily, and a third medication that needs to be taken three times daily; it may not be clear to the patient which times to take each medication are acceptable or optimal. Moreover, one or more additional factors may further complicate the patient's task in scheduling the taking of the medication. For example, each medication may further include additional instructions from the patient's doctor, such as "take with food" or "take at bedtime." In addition, certain medications may negatively interact if taken at or near the same time. Also, each medication may have chromotherapeutic aspects, in which the time of day the medication is taken may increase or decrease its efficacy.

A doctor or pharmacist may be able to aid the patient in scheduling the taking of some of the medication, but many patients receive their prescribed medication from multiple doctors and/or from multiple sources (such as retail pharmacies and mail order pharmacies) and may further take over-the-counter medication and/or natural supplements. A qualified third party, such as a clinician, may be able to aid the patient in optimally planning the timing of taking each medication, but a patient may not have the funds or time for, or even access to, such a third party. An unoptimized medication schedule may not only negatively impact the patient's health, but may also decrease the patient's adherence to the medication regimens because the patient has to remember to take medications at multiple points throughout the day. It is with respect to these and other considerations that the present improvements are desired.

US2011/000170 A1 describes a method for generating a medication regimen of a patient is disclosed. The method comprises receiving a plurality of prescriptions for the patient, wherein a prescription of the plurality of prescriptions indicates a medication and a frequency for administering a dose of the medication. The method further comprises mapping a plurality of doses of one or more medications to a prescription calendar based on the frequencies indicated in the plurality of prescriptions and determining a schedule for administering a plurality of doses of different medications corresponding to each prescription of the plurality of prescriptions based on the calendar and constraints regarding administering the plurality of doses.

### Summary

The matter for protection is defined by the claims.

### Brief Description of the Drawings

By way of example, specific embodiments of the disclosed device will now be described, with reference to the accompanying drawings, in which:
FIG. 1 is a block diagram illustrating a system for automatic medication scheduling in accordance with the present disclosure.
FIG. 2 is a block diagram illustrating an embodiment of an automatic medication scheduling server of the system shown in FIG. 1 in greater detail in accordance with the present disclosure.
FIGS. 3A-C illustrate embodiments of a medication schedule before and after consolidation, in accordance with the present disclosure.
FIGS. 4A-C illustrate embodiments of a second medication schedule before and after conflict resolution, in accordance with the present disclosure.
FIGS. 5-8 are flow diagrams illustrating exemplary methods for automatically generating a medication schedule in accordance with the present disclosure.
FIG. 9 is a block diagram illustrating an embodiment of a centralized system.
FIG. 10 is a block diagram illustrating an embodiment of a distributed system.
FIG. 11 is a block diagram illustrating an embodiment of a computing architecture.
FIG. 12 is a block diagram illustrating an embodiment of a communications architecture.

### Detailed Description

Systems and methods for automatically generating a medication schedule for a patient in accordance with the present disclosure will not be described more fully with reference to the accompanying drawings. In general, the present disclosure provides systems and methods to generate a medication schedule that is as consolidated as possible (i.e., consolidates multiple medication schedules into as few scheduled takings per day, week, and/or month as possible), given drug information and prescription information. The systems and methods also take into account conflict information associated with medication events in the schedule, which may minimize adverse drug administration interactions. In various embodiments, the present invention also takes into account special instructions from a doctor and/or schedules medications at an optimal time of day (i.e., chronotherapeutic adjustments).

It is important to note that the disclosed systems and methods may be embodied in many different forms and should not be construed as limited to the embodiments set forth herein. In the drawings, like numbers refer to like elements throughout.

### Automatic Medication Scheduling Server Overview

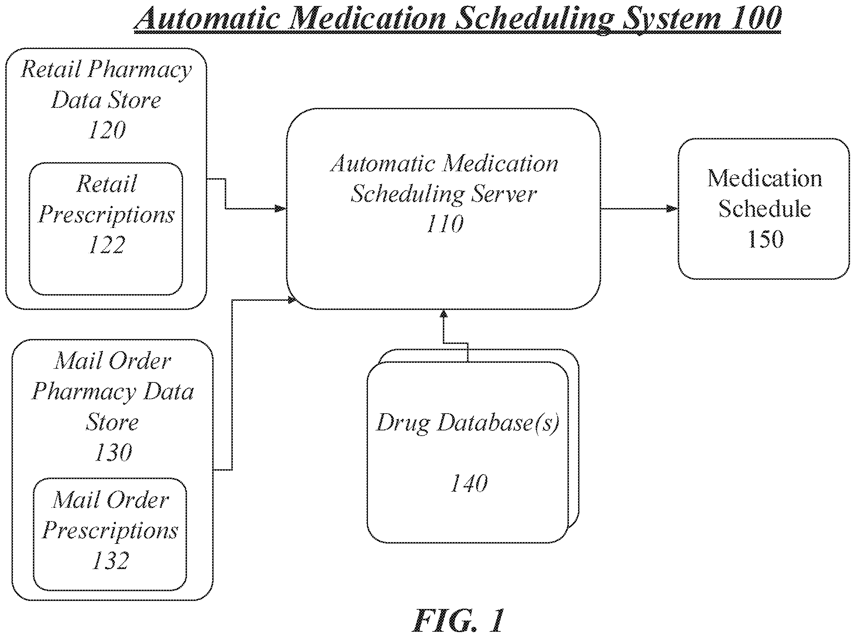

A first exemplary operating environment in accordance with the present disclosure is depicted in FIG. 1. A system 100 includes an automatic medication scheduling server 110 that uses as input prescription information from one or more retail pharmacy data stores 120 and/or one or more mail order pharmacy data stores 130. Any other source of prescription information is within the scope of the present invention, however. The automatic medication scheduling server 110 may receive retail prescriptions 122 and mail order prescriptions 132 for a particular patient and may use the information in the prescriptions to generate and output a medication schedule 150 for a patient. The automatic medication scheduling server 110 consults one or more drug databases 140 to minimize conflicts among medications and, in some examples, to address chronotherapeutic considerations associated with some medications. Conflicts among medications may include any adverse interactions between two or more medications take at or near the same time, and a chronotherapeutic consideration may include any time-related factor that makes a medication more or less effective, or time-related method of reducing the impact of side-effects.

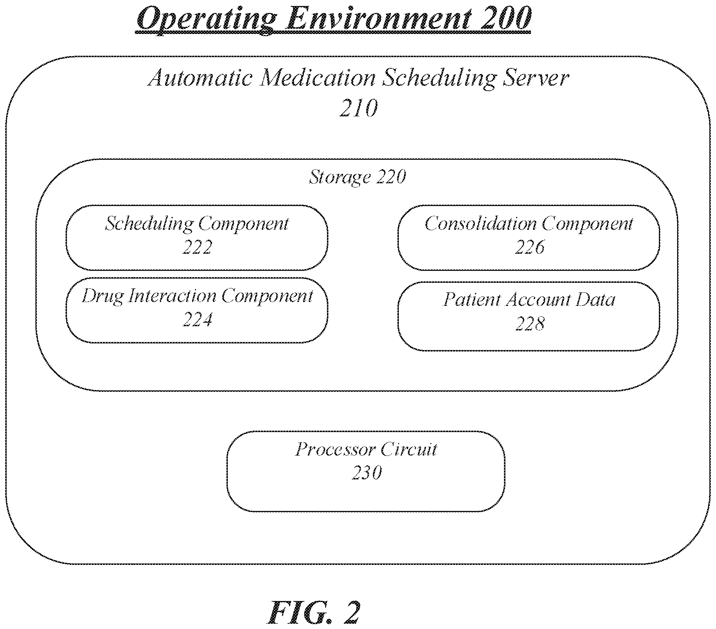

The automatic medication scheduling server 110 may be any of a variety of types of computing devices. For example, without limitation, the automatic medication scheduling server 110 may be a server, a desktop computer, a data entry terminal, a laptop computer, a netbook computer, a tablet computer, a smart phone, or the like. It is important to note that although the automatic medication scheduling server 110 is depicted and discussed herein as a single device, the automatic medication scheduling server 110 may be implemented on multiple devices. The operation of the automatic medication scheduling server 110 are discussed in more detail with respect to FIG. 2.

### Pharmacy Data Stores

The retail pharmacy data store 120 may include servers and/or data stores for any pharmacy entity that operates a brick-and-mortar retail location where a patient can pick up a prescription medication from a pharmacist. Examples of retail pharmacies include, without limitation, CVS Pharmacies, RITE AID Pharmacies, and WALGREEN Pharmacies. A retail pharmacy data store 120 may be accessible to the automatic medication scheduling server 110 over a network, such as the Internet, or over a secured network (e.g., VPN, intranet, or the like).

The mail order pharmacy data store 130 may include servers and data stores for any pharmacy entity that operates an online or other mail order pharmacy. Patients or their prescribers may submit a prescription via mail, telephone, fax, or computer, and the medication is mailed or delivered to an address specified by the patient, such as a home or office address. Examples of mail order pharmacies include, without limitation, MEDCO and EXPRESS SCRIPTS. In some cases, a retail pharmacy may also operate a mail order pharmacy. Some health insurance providers operate their own mail order pharmacies.

Specialty pharmacies (not shown) may also provide the services of retail and/or mail order pharmacies within the system 100. Specialty pharmacies may fill prescriptions for medications or treatments that are not typically available at a retail or mail-order pharmacy.

A prescription, generally, may include a medication name, a medication dosage, information about a time and frequency to take the medication, other instructions for use, a number of refills, a prescribing physician's name, and/or an expiration date beyond which the prescription may not be filled or refilled. Retail prescriptions 122 may include some or all prescriptions submitted to the retail pharmacy for a given patient. Mail order prescriptions 132 may include some or all prescriptions submitted to the mail order pharmacy for a given patient.

### Drug Database(s)

Drug database(s) 140 may include data stores that include information about prescription and over-the-counter medications. In particular, the drug databases 140 include conflict information such as information about adverse drug interactions for one medication with respect to other medications, information about increased or decreased effectivenesss of a medication when taken with another medication (i.e., chronotherapy), and/or possible side effects of a medication.

The drug database(s) 140 may include commercial databases, such as, without limitation, FIRST DATABANK (FDB), and MEDISPAN. The drug database(s) 140 may include proprietary or in-house databases maintained by pharmacies or drug manufacturers. The drug database(s) 140 may include databases operated by a government agency, such as the Federal Drug Administration.

The drug database(s) 140 may be accessed by using industry standard drug identifiers, such as and without limitation, a generic product identifier (GPI), national drug code directory (NDC), universal product code (UPC), health related item (HRI), or manufacturer (MFR). The clinical attributes of the drugs may be gathered from the various drug database(s) 140 and the information may be selectively parsed. In some embodiments, the information from commercial drug databases may be stored in in-house databases. The stored data may be synced between commercial and in-hose databases using batch or real time updates. The embodiments are not, however, limited only to these examples.

### Medication Scheduling

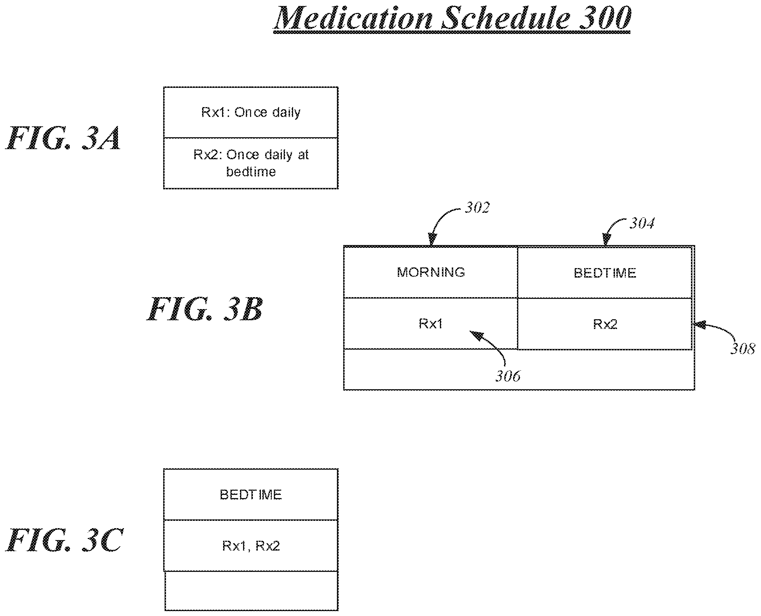

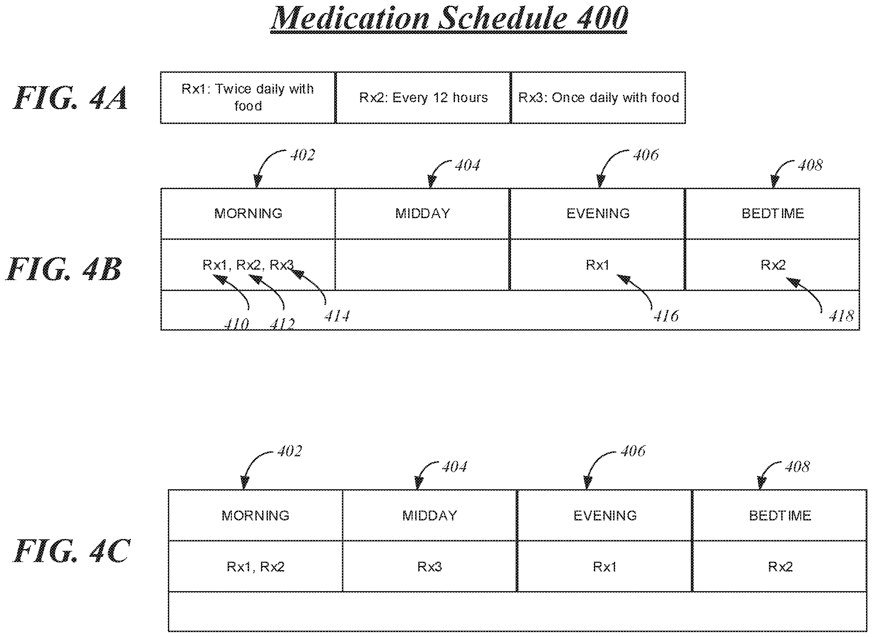

The medication schedule 150 output by the automatic medication scheduling server 110 may be a digital file stored on a computer. The medication schedule 150 includes plural time slots; in one embodiment, the medication schedule includes four time slots, but the present invention is not limited to any particular number of time slots. Generally a medication schedule 150 comprises a plurality of days each divided into at least two time slots. For instance, the medication schedule 150 may represent a week or a month. Prescribed medications are allocated to time slots according to the time and frequency information in the prescription instructions for each medication. The automatic medication scheduling server 110 may allocate the prescriptions to the time slots according to the drug information, the prescription information, chronotherapeutic considerations, and default slot rules, and then resolves conflicts and consolidate medications into as few time slots as possible before outputting the mediation schedule 150. The medication schedule 150 is output in electronic form. For instance, the medication schedule 150 may be output to the patient or may be submitted to the pharmacies used by the patient to update their respective prescription data for the patient. In some examples, the medication schedule may be provided to the patient in paper form. Examples of mediation schedules are described with respect to FIGS. 3 and 4.

### Automatic Medication Scheduling Server Details

FIG. 2 is a block diagram of an operating environment 200 that includes an embodiment of an automatic mediation scheduling server 210. The automatic medication scheduling server 210 may be an example of the automatic medication scheduling server 110. In various embodiments, the automatic medication scheduling server 210 incorporates one or more processor circuits 230 and at least one storage 220. The storage 220 may include any volatile or non-volatile computer-readable storage medium, and is not intended to include electro-magnetic signals, optical signals, or other carrier waves. The storage 220 stores one or more functional components, such as, but not limited to, a scheduling component 222, a drug interaction component 224, and a consolidation component 226. The storage 220 may also store patient account data 228. In the automatic medication scheduling server 210, the functional components may correspond to ta sequence of instructions operative on the processor circuit 230 to implement logic to perform various functions, as will be described in further detail. Although the functional components are shown and described herein in a particular configuration and number, embodiments may include more, fewer, or other components to provide the same or similar functionality.

In an embodiment, a patient may register with the automatic medication scheduling server 210. Registering may create a patient account that is stored in patient account data 228. The patient account data 228 may include patient identifying information that may be used by the scheduling component 222 to obtain the patient's prescription information from the respective pharmacy data stores. For example, the patient account data 228 may include an electronic medical record identifier, a health insurance plan account identifier, a social security number, or any other means to identify all of the patient's prescription information across the pharmacies used by the patient. In an embodiment, the patient may need to provide consent to allow the scheduling component 222 to access prescriptions 122 and 132. Other embodiments may not require patient registration. The automatic medication scheduling server 210 may have access to all of a patient's prescriptions across a pharmacy enterprise once a patient has at least one prescription filled by a pharmacy within the pharmacy enterprise.

The scheduling component 222 generates an initial medication schedule for a patient. The scheduling component 222 may request or retrieve the retail prescriptions 122 and the mail order prescriptions 132 for a patient from the retail pharmacy data store 120 and the mail order pharmacy data store 130, respectively.

The scheduling component 222 may inspect each prescription and interpret the prescription information that accompanies the medication name and dosage. For example, the information may include a physician instruction to "take once daily," to "take twice a day with food," to "take every 4 hours," or to "take at bedtime." The scheduling component 222 may use natural language processing and/or look for keywords to determine a frequency for a dose of medication.

The scheduling component 222 may create one or more medication events for a prescription. A medication event comprises a mediation and a dosage, which may include a unit of measure, e.g. an amount in milligrams, or a number of tablets or pills. For example, the scheduling component 222 may create four medication events for a mediation that needs to be taken four times a day. Each medication event may be allocated to a time slot. A time slot may correspond to some portion of the schedule period, e.g., morning, midday, evening, or bedtime. A time slot may correspond to a particular clock time, e.g. 8:00 a.m.

Initially, the scheduling component 222 may allocate all medication events according to any specific instructions, such as "at bedtime". For medication events with an unspecified time of day, the scheduling component 222 may apply default rules to allocate medication events. For example, a "once daily" medication event may be allocated to the "morning" slot automatically. A medication event that occurs twice a day without further specification may be allocated to time slots spaced evenly apart, such as in "morning" and "bedtime". When additional instructions are present, e.g. "take with food", the scheduling component 222 may apply rules to allocate medication events to time slots associated with meals, e.g. morning, middayor evening.

### Chronotherapy

Once an initial medication schedule is generated, the scheduling component 222 may resolve any chronotherapeutic considerations not already addressed by following specific prescriber instructions. For example, the scheduling component 222 may consult the drug database(s) 140 to determine whether a medication is more effective at certain times of day, or may cause undesirable side effects, such as drowsiness, insomnia, or dizziness. If, for example, a medication event allocated to a morning time slot causes drowsiness, the scheduling component 222 may then move the mediation event to a "bedtime" time slot in response to receiving the chronotherapeutic considerations from the drug databases 140.

### Drug Administration Interactions

The scheduling component 222 may pass the medication schedule to the drug interaction component 224 to attempt to resolve any conflicts present in the mediation schedule. The drug interaction component 224 examines the medication events in each time slot and consult with the drug databases 140 to determine whether two or more medications in a time slot, or within a time period of another medication event in another time slot, conflict. A conflict may include an adverse drug interaction, e.g. negative side effects caused by taking two or more mediations together or too close together in time, and/or drug administration interactions. A conflict may include a change (increase or decrease) in medication effectiveness caused by taking two or more medications together or too close together in time.

The drug interaction component 224 iteratively examines each medication event in each time slot and, when a conflict is detected, moves one of the medication events to a different time slot. When selecting which conflicting medication event to move, the drug interaction component 224 may move the medication event having fewer specifications from the prescription information. For example, a medication having only a "take once daily" instruction may be moved if the conflicting mediation has a "take once daily on an empty stomach" instruction.

The scheduling component 222 and the drug interaction component 224 iterates over their respective functions for each medication in each time slot until no medications are moved, indicating that all conflicts are resolved. In the event that a conflict cannot be resolved, a predetermined number of iterations is used to prevent an endless loop. An alert may be issued to have a human operator intervene, for example by substituting a different medication, or by identifying a clinically appropriate time slot based on clinical judgment.

### Consolidation

Once the chronotherapeutic considerations and conflicts are addressed, the consolidation component 226 examines the medication schedule and determines whether the total number of time slots used in the medication schedule can be reduced.

The consolidation component 226 examines time slots having only one medication event allocated to them. If a medication event in a time slot has an unspecified time requirement, it may be movable to another time slot that already has medication events allocated to it. For example, if a mediation event is scheduled for "once daily" but the time of day is unspecified, it may be moved from a default morning slot to another time slot. When a movable medication event is identified, the consolidation component 226 identifies a candidate time slot to allocate to the moveable medication event. The consolidation component 226 determines, for instance by coordinating with the drug interaction component, whether the movable medication event will conflict with the medication events in the candidate time slot. If there is no conflict, the movable medication event is be moved to the candidate time slot, thus reducing the number of different time slots needed in the medication schedule 150.

### Examples

FIGS. 3A-3C are block diagrams that, collectively, illustrate an exemplary medication schedule 300 before and after consolidation by the consolidation component 226.

FI**G**. **3**A illustrates two prescriptions for a patient. One prescription is for the medication "Rx1" which has the instruction to be taken "once daily". The second prescription is for the medication "Rx2" which has the instruction to be taken "once daily at bedtime".

**F**IG. **3**B illustrates the medication schedule 300 prior to consolidation. In some embodiments, the scheduling component 222 and the drug interaction component 224 may have already operated on the two prescriptions shown in FIG. 3A to generate the medication schedule 300. The medication schedule 300 as shown in FIG. 3B includes two time slots: a morning time slot 302 and a bedtime time slot 304; and two medication events: medication event 306 for "Rx1" and medication event 308 for "Rx2." The medication event 306 has been allocated to the morning time slot 302, and the medication event 308 has been allocated to the bedtime time slot 304. As shown, this version of the medication schedule 300 requires that the patient remember to take one medicine in the morning and another at bedtime.

**F**IG. **3**C illustrates the medication schedule 300 after consolidation. The consolidation component 226 may have examined the morning time slot 302 and found a medication event 306 that had an unspecified time component. The consolidation component 226 may have looked for a second time slot that already had a medication event allocated to it, and found the bedtime time slot 304. After checking that the medication event 306 did not conflict with the medication event 308, the consolidation component 226 moved the medication event 306 to the bedtime time slot 304, thus reducing the number of time slots in use in the medication schedule 300. As shown in FIG. 3C, the patient now only needs to remember to take medications once a day

**F**IGS. **4**A-4C are block diagrams that, collectively, illustrate a medication schedule 400 before and after resolving conflicts and chronotherapeutic considerations by the scheduling component 222 and the drug interaction component 224.

**F**IG. **4**A illustrates three prescriptions for a patient. One prescription is for the medication "Rx1" which has the instruction from a drug database 140 to be taken "twice daily with food". The second prescription is for the medication "Rx2" which has the instruction from a drug database 140 to be taken "every twelve hours". The third prescription is for the medication "Rx3" which has the instruction from a drug database 140 to be taken "once daily with food." In some embodiments, the instructions may also or alternatively be a part of the physician provided instructions for a prescription.

**F**IG. **4**B illustrates the medication schedule 400 after a default scheduling operation and prior to resolving conflicts and chronotherapeutic considerations. The medication schedule 400 as shown in FIG. 4B includes four time slots: a morning time slot 402, a midday time slot 404, an evening time slot 406, and a bedtime time slot 408. Each time slot may have some associated parameters, such as whether food consumption is possible during the time slot, and how far apart in time each time slot may be from the other time slots. The medication schedule 400 in FIG. 4B also has five medication events: medication events 410 and 416 for "Rxl", medication events 412 and 418 for "Rx2", and medication event 414 for "Rx3".

In an embodiment, the medication schedule 400 as shown in FIG. 4B may have been generated as follows. For the medication Rx1, the scheduling component 222 may normally interpret the instruction of "twice daily" as twelve hours apart, but the added instruction of "with food" may eliminate the bedtime time slot 408 from consideration, resulting in allocating the Rx1 medication events 410 and 416 to the morning time slot 402 and evening time slot 406, respectively. Similarly, the scheduling component 222 may interpret the instruction for medication Rx2 either as actually 12 hours apart, or as far apart from each other as possible within the medication schedule 400. Accordingly, the medication events 412 and 418 are allocated to the morning time slot 402 and the bedtime time slot 408, respectively. Finally, the instruction of "once daily" for Rx3 may normally result in a default placement in the morning time slot 402. Since the morning time slot 402 may be associated with a food event, i.e. breakfast, the additional instruction of "with food" does not change the allocation.

**F**IG. **4**C illustrates the medication schedule 400 after conflicts are resolved. For the purpose of illustration, assume that medication Rx3 conflicts with Rx1. The drug interaction component 224 may examine the morning time slot 402 and may determine whether Rx1, Rx2 and Rx3 conflict with each other. When the drug interaction component 224 determines that Rx1 and Rx3 conflict with each other, the drug interaction component 224 may select which medication event to move to resolve the conflict. In some embodiments, medications are selected to be moved based on their chronological order of filling; in other embodiments, a medication having fewer medication events may be easier to move, and may be selected first in attempting to resolve the conflict, Any method of selecting medications to be moved is, however, within the scope of the present invention. Accordingly, the drug interaction component 224 may select Rx3 to move. The "once daily" instruction means that Rx3 may potentially be allocated to any other time slot. However, the additional instruction of "with food" eliminates the bedtime time slot 408 from consideration. The evening time slot 404 also contains a medication event for Rx1, eliminating it from consideration. The only remaining available time slot is the midday time slot 404. The drug interaction component 224 may therefore allocate the medication event 414 for Rx3 to the midday time slot 404 to resolve the conflict. In an embodiment, the drug interaction component 224 may instruct the scheduling component 222 to move the medication event 414 for Rx3 to the midday time slot 404, rather than performing the action itself.

The medication schedules 300 and 400 as shown in FIGS. 3 and 4 are provided for illustration purposes only. The embodiments are not limited to the examples presented herein. Medication schedules with more or fewer time slots are possible.

Included herein is a set of flow charts representative of exemplary methodologies for performing novel aspects of the disclosed architecture. While, for purposes of simplicity of explanation, the one or more methodologies shown herein, for example, in the form of a flow chart or flow diagram, are shown and described as a series of acts, it is to be understood and appreciated that the methodologies are not limited by the order of acts, as some acts may, in accordance therewith, occur in a different order and/or concurrently with other acts from that shown and described herein. For example, those skilled in the art will understand and appreciate that a methodology could alternatively be represented as a series of interrelated states or events, such as in a state diagram. Moreover, not all acts illustrated in a methodology may be required for a novel implementation.

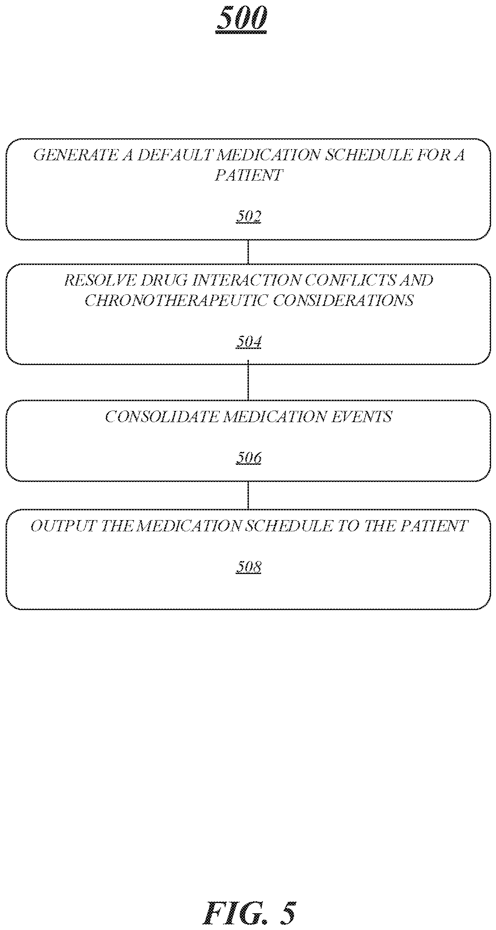

FIG. 5 illustrates a logic flow 500. The logic flow 500 may be representative of some or all of the operations executed by one or more embodiments described herein. In particular, the logic flow 500 may represent an overview of the operations executed by the system 100 in creating a medication schedule 150.

The logic flow 500 generates a default medication schedule for a patient in block 502. For example, the scheduling component 222 may use the prescription information for a patient to create a medication schedule having at least two time slots (in some embodiments, four time slots) per day, and may allocate medication events to the time slots.

The logic flow 500 resolves drug interaction conflicts and, in some examples, chronotherapeutic considerations in block 504. For example, the scheduling component 222 may examine medication events placed by default rule for any chronotherapeutic considerations. A chronotherapeutic consideration may include any time-related factor that makes a medication more effective (i.e., by increasing the intended effects on the patient of taking the medication) or any time-related method of reducing the impact of side effects (i.e., by decreasing any undesirable effects on the patent of taking the medication). For example, a medication that was placed by default in a morning time slot might cause drowsiness or dizziness and be recommended for bedtime, according the drug databases 140. As another example, a medication that was placed by default in a morning time slot might be more effective or better tolerated if taken in the evening. Accordingly, the scheduling component 222 may move the medication event to a bedtime time slot.

The drug interaction component 224 examines medication events in the same time slot, or medication events in adjacent time slots, for conflicts, and moves one or more medication events to different time slots to resolve a conflict.

The logic flow 500 consolidates medication events in block 506. The consolidation component 226 examines the time slots having only one medication event to determine whether that time slot can be emptied of medication events by moving the medication event(s) to a time slot already having one or more medication events, without causing medication conflicts or violating chronotherapeutic considerations.

The logic flow 500 outputs the medication schedule, for instance to the patient block 508. Once the medication schedule is consolidated and has minimized conflicts, the medication schedule 150 is output, for instance to the patient. For example, the medication schedule 150 may be printed and provided to the patient when a prescription on the medication schedule 150 is picked up or mailed. The medication schedule 150 may be emailed to the patient, posted on an online health care portal accessible to the patient by a web browser, or added to an electronic medical record for the patient. In some embodiments, the medication schedule 150 may also or alternatively be sent to the pharmacies and to the prescribers of the patient. The output medication schedule may also include information about medications and/or other therapies that were not allocated to the time slots of the medication schedule. In some embodiments, the portion of the medication schedule 150 relevant to a specific medication, e.g. a prescription dose calendar, may be printed on a label and affixed to a prescription container for that medication.

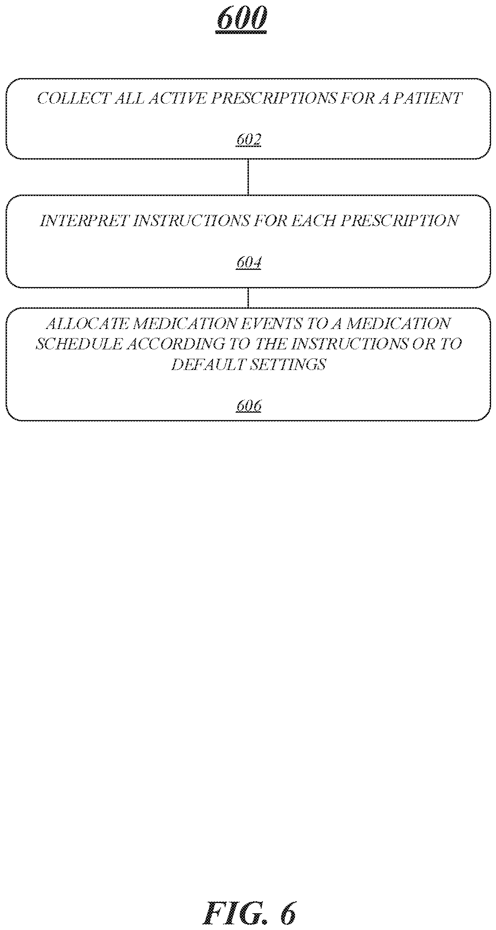

FIG. 6 illustrated a logic flow 600. The logic flow 600 may be representative of some or all of the operations executed by one or more embodiments described herein. In particular, the logic flow 600 may represent a more detailed view of the block 502 from the logic flow 500.

The logic flow 600 may collect all of the active prescriptions for a patient in block 602. For example, the scheduling component 222 may collect all of the active prescriptions for the patient from the pharmacy data stores 120, 130. Active prescriptions may include prescriptions that are not discontinued, that are prescribed but not yet filled (which may include prescriptions that are "on hold"), that have been filled at least once, and/or that have unused refills that have not expired. In some embodiments, duplicate prescriptions may be removed.

The logic flow 600 may interpret the instructions for each prescription in block 604. For example, the scheduling component 222 may use natural language processing or keyword matching to interpret the instructions for each prescription and create medication events according to the instructions. The scheduling component 222 may determine how many times in a day (or other schedule period) a medication should be taken, and may create a medication event for each time that the medication should be taken. The scheduling component 222 may further determine if there are any additional restrictions on a medication event, such as whether the medication should be taken with or without food, or only at night or in the morning. Table 1 illustrates some examples of default rules for allocating medication events to time slots according to the instructions.

**Table 1**

| Instruction | Default Time Slots |
|---|---|
| Four times daily | Morning, Midday, Evening, Bedtime |
| Every six hours | Morning, Midday, Evening, Bedtime |
| Three times daily; or Every eight hours | Morning, Midday, Bedtime |
| Three times daily with food; or Every eight hours with food | Morning, Midday, Evening |
| Two times daily; or Every twelve hours | Morning, Bedtime |
| Two times daily with food; or Every twelve hours with food | Morning, Evening |
| Once daily; or every 24 hours | Morning |

The logic flow 600 may allocate one or more medication events to a medication schedule in block 606. For example, the scheduling component 222 may allocate the medication events to a medication schedule according to the instructions and any default rules. For example, when instructions specify both a number of dosage events and a timing instruction, e.g. in the morning and at bedtime, the scheduling component 222 may allocate the medication events simply according to the instructions. When the instructions provide a frequency or a number of dosage events without a timing instruction, the scheduling component 222 may use a default rule to allocate the first medication event to the first time slot in the medication schedule, and may allocate subsequent medication events for that prescription according to the frequency or the number of events distributed across the medication schedule as evenly as possible.

In some embodiments, some prescriptions may be included on the medication schedule, e.g. in a separate section, but not allocated to a time slot. For example, medications that have a variable frequency, e.g. every 6 to 8 hours, and medications that have a variable dose over time may not be slotted. Other prescriptions that may or may not be slotted include medications taken only once; taken "as needed"; taken on a non-daily basis (unless the medication schedule has time slots spanning more than one day); taken at more times than there are time slots in the medication schedule; and/or taken at specific times of the day.

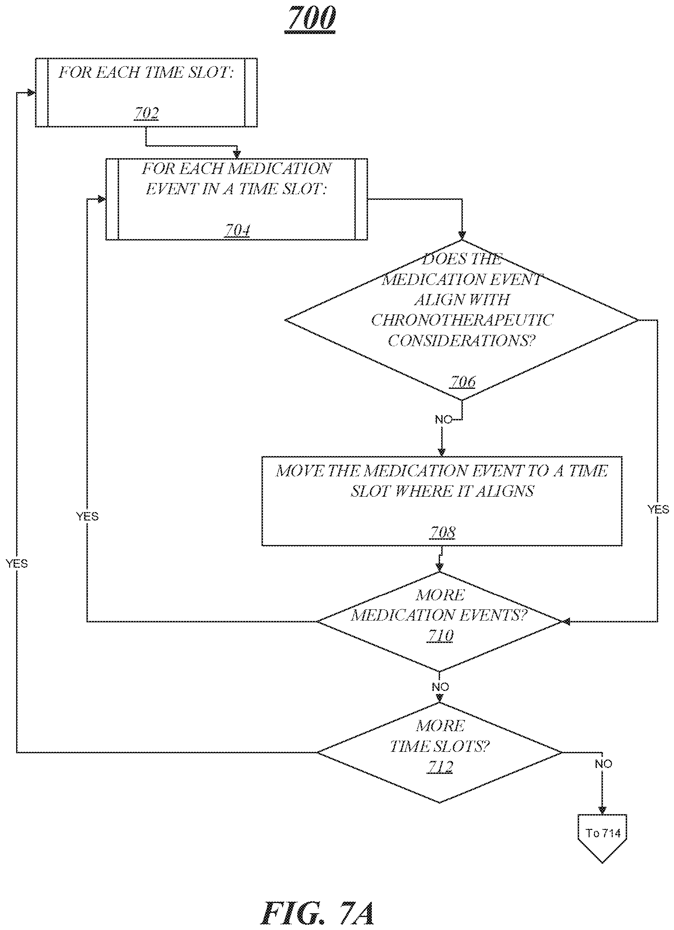

**FIGS. 7A-7B** illustrates a logic flow 700. The logic flow 700 may be representative of some or all of the operations executed by one or more embodiments described herein. In particular, the logic flow 700 may represent a more detailed view of the block 504 from the logic flow 500.

The logic flow 700 may iterate over each time slot in the medication schedule beginning at block 702. In each time slot, the logic flow 700 may iterate over each medication event beginning at block 704.

The logic flow 700 may, for a given medication event, determine whether the medication event aligns with any chronotherapeutic considerations at block 706. For example, the scheduling component 222 may query the drug database(s) 140 to determine whether there is a preferred or recommended time of day for taking the medication. If a preferred or recommended time of day exists for the medication and the medication event is not already scheduled for a time slot corresponding to that time of day, the logic flow 700 may, in block 708, move the medication event to a time slot where the medication event does align with the chronotherapeutic considerations. For example, for a medication that causes insomnia, the medication event may be moved from an evening or bedtime time slot to a morning time slot.

In some embodiments, the drug database(s) 140 additionally contain information regarding a non-preferred or non-recommended time of day for taking the medication. In these embodiments, the logic flow 700 moves the medication event to a time slot different from the non-preferred time slot.

Once any preferred or non-preferred times of day for the medication event have been considered in block 706 and potentially moved in block 708, the logic flow 700 may proceed to determining whether there is another medication event to examine in the time slot at block 710.

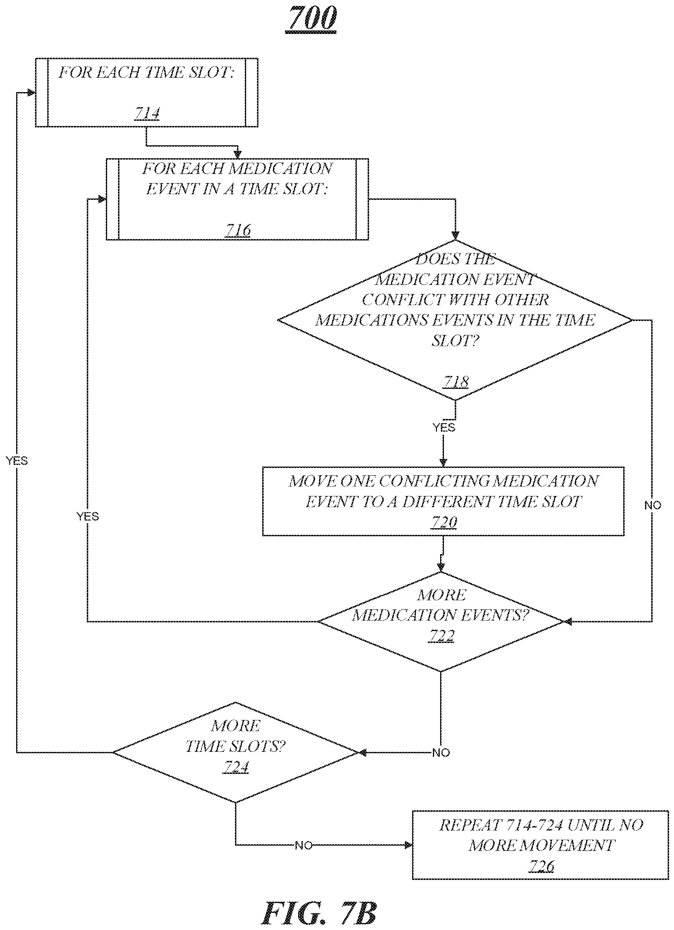

The logic flow 700 may determine whether there are any remaining unexamined medication events in the time slot in block 710. When there are still unexamined medication events, the logic flow 700 may repeat blocks 704 to708. When there are no remaining unexamined medication events in the time slot, the logic flow 700 may determine whether there are any remaining time slots to check, in block 712. When there are unexamined time slots, the logic flow 700 may repeat blocks 702 to 710. When all of the medication events in all of the time slots have been examined, the logic flow 700 may proceed to block 714 in FIG. 7B.

The logic flow 700 may continue in FIG. 7B, and may again iterate over each time slot, beginning with block 714. In each time slot, the logic flow 700 iterates over each medication event in the time slot, beginning at block 716.

The logic flow 700, for a given medication event, determines whether the medication event conflicts with another medication event in the time slot at block 718. For example, the drug interaction component 224 queries the drug database(s) 140 to determine whether the medication of the medication event has conflicts with other medications. If the drug databases 140 return a list of conflicting medications, the drug interaction component 224 may compare the other medication events in the time slot to the list to identify conflicts. The drug interaction component 224 may, alternatively, query the drug databases 140 to determine whether the medication of the medication event has conflicts specifically with the other medications in the same time slot or in an adjacent time slot.

When the medication event does have a conflict, the logic flow 700 moves one of the conflicting medication events to a different time slot at block 720. The medication event that is moved may be different from the medication event selected for the current iteration of the logic flow. For example, the drug interaction component 224 or the scheduling component 222 may select the medication event having the fewest limiting instructions to move.

The logic flow 700 may determine whether there are any remaining unexamined medication events in the time slot in block 722. When there are no remaining unexamined medication events in the time slot, the logic flow 700 may determine whether there are any remaining time slots to check, in block 724. When there are unexamined time slots, the logic flow 700 may repeat block 714 to 722.

When all of the medication events in all of the time slots have been examined, the logic flow 700 proceeds to block 726, where blocks 714 to 724 are repeated until no medications events are moved.

In the event that a conflict cannot be resolved, or when resolving one conflict creates another conflict, the logic flow 700 includes a limit on the number of repetitions of block 726, and may issue an alert to a human operator that the conflict(s) cannot be resolved (not shown). For example, if all conflicts are not resolved in two, three, or four repetitions, the logic flow 700 may halt further attempts to resolve conflicts. In other embodiments, the logic flow 700 halts further attempts to resolve conflicts if it detects that each additional repetition is merely moving the same medication or medications back-and-forth between different time slots. In some embodiments, the logic flow 700 may use information from the drug databases 140 to suggest an alternate medication that, if substituted for a conflict-causing medication, may resolve the conflict.

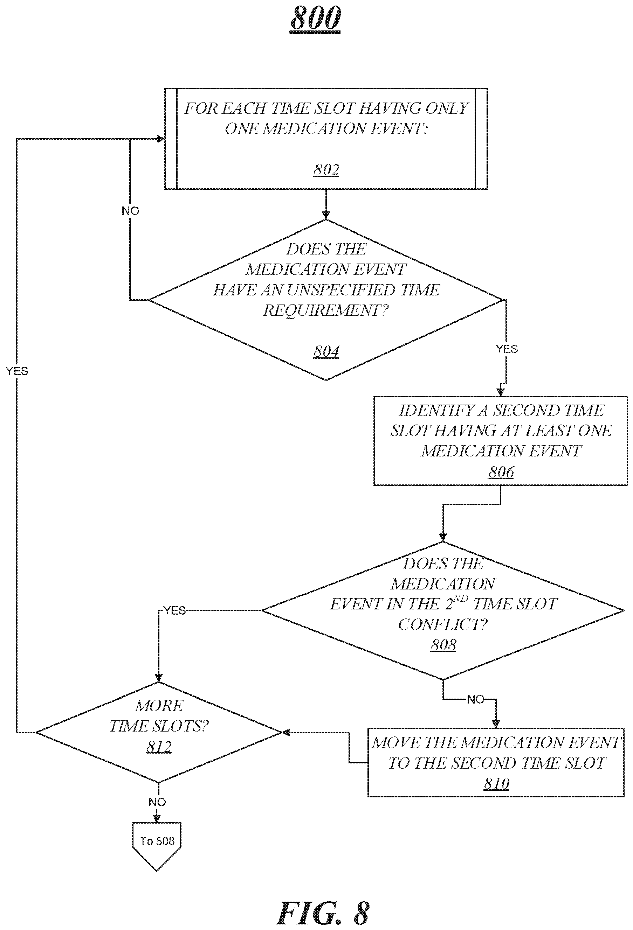

FIG. 8 illustrates a logic flow 800. The logic flow 800 may be representative of some or all of the operations executed by one or more embodiments described herein. In particular, the logic flow 800 may represent a more detailed view of the consolidation block 506 from the logic flow 500.

The logic flow 800 may iterate over some or all of the time slots in the medications schedule, beginning in block 802. In an embodiment, the logic flow 800 may iterate over the time slots having only one medication event allocated to them. In other embodiments, the logic flow 800 may iterate over time slots having a relatively smaller number of medication events allocated thereto, or over all of the time slots having medication events.

The logic flow 800 may determines whether the medication event in the given time slot (referred to as the first medication event) has an unspecified time requirement in block 804. For example, the consolidation component 226 may determine if the medication event has any instructions that specify a number of frequency of dosages without limiting the dose to a particular time of day. For example, a medication event having the instruction "once daily" or "twice daily" may have an unspecified time requirement.

When the first medication event has an unspecified time requirement, the logic flow 800 identifies a second time slot in the medication schedule that has at least one medication event allocated to in block 806. If there are no other time slots having medication events, then the logic flow 800 may end (not shown).

The logic flow 800 determines whether the first medication event conflicts with the medication event(s) in the second time slot, in block 808. For example, the consolidation component 226 determines whether a conflict exists, for instance by invoking the drug interaction component 224 or querying the drug databases 140 directly to determine whether a conflict exists.

The logic flow800 moves the first medication event to the second time slot in block 810 when no conflict exists. If there is a conflict, then the first medication event may be left in its allocated time slot.

The logic flow 800 may determine whether there are any time slots remaining to examine for consolidation at block 812. If there are time slots remaining, the logic flow 800 may repeat blocks 802 to 812 until there are no more time slots to examine.

The logic flow 800 may return to block 508 of the logic flow 500 when no time slots remain to be consolidated.

While illustrated separately herein, the logic flows illustrated in FIGS. 5-8 may be combined in part or in whole. Further, the operations illustrated in FIGS. 5-8 may occur serially or in parallel. As a result of the logic flow 500, as implemented with the logic flows 600, 700, and/or 800, or in other implementations, a medication schedule is created for a patient that minimizes medication conflicts and that has as few time slots as possible. In some embodiments, chronotherapeutic effects of the medication are taken into account, and the mediation schedule is adjusted accordingly.

In some embodiments, feedback from a caregiver or the patient may prompt a re-allocation of medication events to a patient's medication schedule, and may introduce additional constraints, for example, if the patient is unable or unwilling to take a medication in a specific time slot, or is experiencing unexpected side effects or drug interactions.

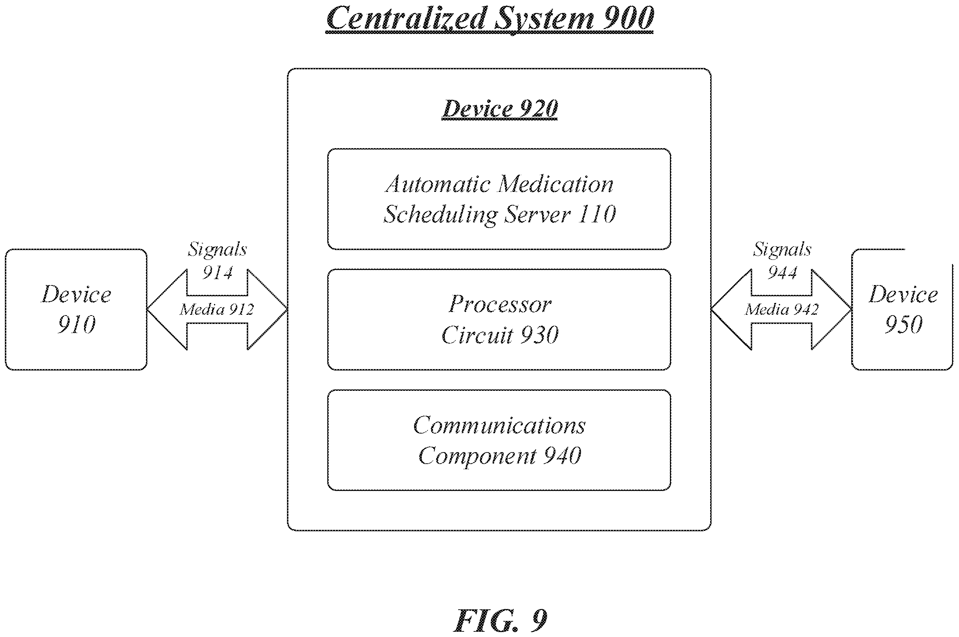

**FIG. 9** illustrates a block diagram of a centralized system 900. The centralized system 900 may implement some or all of the structure and/or operations for the system 900 in a single computing entity, such as entirely within a single device 920.

The device 920 may comprise some or all of the components of the automatic medication scheduling server 110, and may also include a processor circuit 930 and a communications component 940.

The device 920 may execute communications operations or logic for the system 900 using communications component 940. The communications component 940 may implement any well-known communications techniques and protocols, such as techniques suitable for use with packet-switched networks (e.g., public networks such as the Internet, private networks such as an enterprise intranet, and so forth), circuit-switched networks (e.g., the public switched telephone network), or a combination of packet-switched networks and circuit-switched networks (with suitable gateways and translators). The communications component 940 may include various types of standard communication elements, such as one or more communications interfaces, network interfaces, network interface cards (NIC), radios, wireless transmitters/receivers (transceivers), wired and/or wireless communication media, physical connectors, and so forth. By way of example, and not limitation, communication media 912, 942 include wired communications media and wireless communications media. Examples of wired communications media may include a wire, cable, metal leads, printed circuit boards (PCB), backplanes, switch fabrics, semiconductor material, twisted-pair wire, co-axial cable, fiber optics, a propagated signal, and so forth. Examples of wireless communications media may include acoustic, radio-frequency (RF) spectrum, infrared and other wireless media.

The device 920 may communicate with other devices 910, 950 over communications media 912, 942, respectively, using communications signals 914, 944, respectively, via the communications component 940. The devices 910, 950 may be internal or external to the device 920 as desired for a given implementation. Devices 910, 950 may include, for example, client computing devices used in a pharmacy, a hospital, or a physician's office.

**F**IG. **1**0 illustrates a block diagram of a distributed system 1000. The distributed system 1000 may distribute portions of the structure and/or operations for the system 100 across multiple computing entities. Examples of distributed system 1000 may include without limitation a client-server architecture, a 3-tier architecture, an N-tier architecture, a tightly-coupled or clustered architecture, a peer-to-peer architecture, a master-slave architecture, a shared database architecture, and other types of distributed systems. The embodiments are not limited in this context.

The distributed system 1000 may comprise a server device 1010 and a server device 1050. In general, the server devices 1010, 1050 may be the same or similar to the automatic medication scheduling server 210 and/or the device 920. For instance, the server device 1010 and the server device 1050 may each comprise a processor circuit 1030 and a communications component 1040 which are the same or similar to the processor circuit 230, 1030 and the communications component, respectively, as described with reference to FIGS. 2 and 9. In another example, the devices 1010, 1050 may communicate over a communications media 1012 using communications signals 1014 via the communications components 1040.

The server device 1010 may comprise or employ one or more programs that operate to perform various methodologies in accordance with the described embodiments. In one embodiment, for example, the server device 1010 may implement the scheduling component 222.

The server device 1050 may comprise or employ one or more programs that operate to perform various methodologies in accordance with the described embodiments. In one embodiment, for example, the server device 1050 may implement at some or all of the remaining components shown in the automatic medication scheduling server 210. The server device 1010 and the server device 1050 may request and receive drug information from the drug databases 140, and other data from each other and/or from the pharmacy data stores 120, 130.

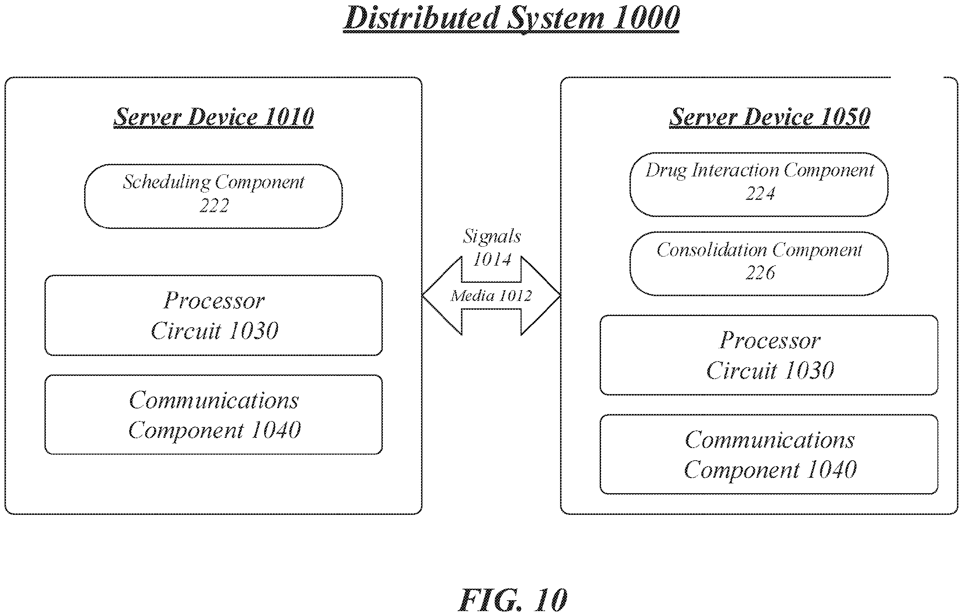

**F**IG. **1**1 illustrates an embodiment of an exemplary computing architecture 1100 suitable for implementing various embodiments as previously described. In one embodiment, the computing architecture 1100 may comprise or be implemented as part of an electronic device. Examples of an electronic device may include those described with reference to FIGS. 1, 2, and 9-10, among others. The embodiments are not limited in this context.

As used in this application, the terms "system" and "component" are intended to refer to a computer-related entity, either hardware, a combination of hardware and software, software, or software in execution, examples of which are provided by the exemplary computing architecture 1100. For example, a component can be, but is not limited to being, a process running on a processor, a processor, a hard disk drive, multiple storage drives (of optical and/or magnetic storage medium), an object, an executable, a thread of execution, a program, and/or a computer. By way of illustration, both an application running on a server and the server can be a component. One or more components can reside within a process and/or thread of execution, and a component can be localized on one computer and/or distributed between two or more computers. Further, components may be communicatively coupled to each other by various types of communications media to coordinate operations. The coordination may involve the unidirectional or bi-directional exchange of information. For instance, the components may communicate information in the form of signals communicated over the communications media. The information can be implemented as signals allocated to various signal lines. In such allocations, each message is a signal. Further embodiments, however, may alternatively employ data messages. Such data messages may be sent across various connections. Exemplary connections include parallel interfaces, serial interfaces, and bus interfaces.

The computing architecture 1100 includes various common computing elements, such as one or more processors, multi-core processors, co-processors, memory units, chipsets, controllers, peripherals, interfaces, oscillators, timing devices, video cards, audio cards, multimedia input/output (I/O) components, power supplies, and so forth. The embodiments, however, are not limited to implementation by the computing architecture 1100.

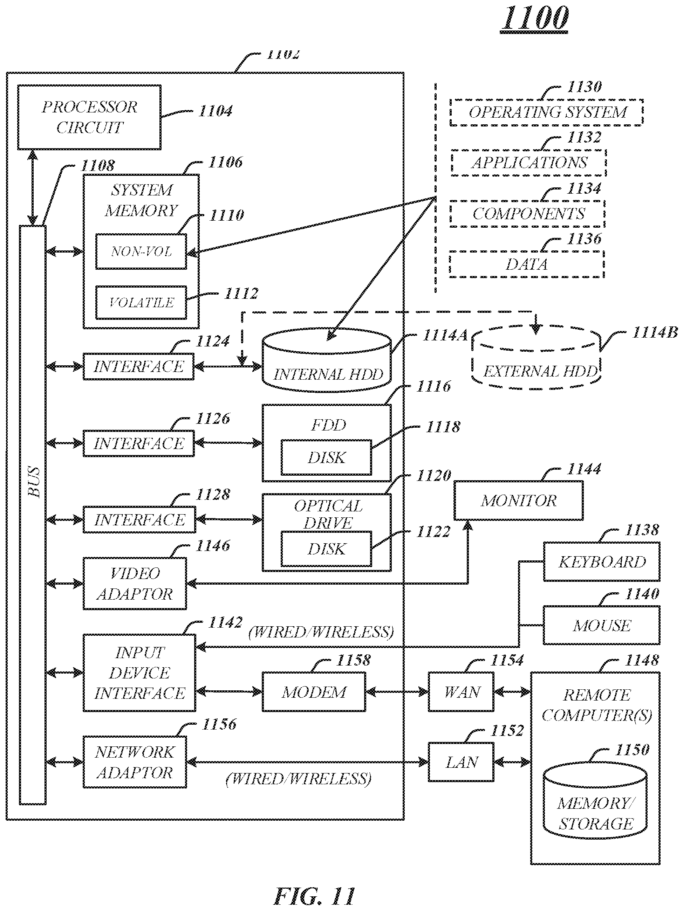

As shown in FIG. 11, the computing architecture 1100 comprises one or more processor circuits 1104, a system memory 1106 and a system bus 1108. The processor circuit 1104 can be any of various commercially available processors, including without limitation an AMD® Athlon®, Duron® and Opteron® processors; ARM® application, embedded and secure processors; IBM® and Motorola® DragonBall® and PowerPC® processors; IBM and Sony® Cell processors; Intel® Celeron®, Core (2) Duo®, Itanium®, Pentium®, Xeon®, and XScale® processors; and similar processors. Dual microprocessors, multi-core processors, and other multiprocessor architectures may also be employed as the processor circuit 1104.

The system bus 1108 provides an interface for system components including, but not limited to, the system memory 1106 to the processor circuit 1104. The system bus 1108 can be any of several types of bus structure that may further interconnect to a memory bus (with or without a memory controller), a peripheral bus, and a local bus using any of a variety of commercially available bus architectures. Interface adapters may connect to the system bus 1108 via a slot architecture. Example slot architectures may include without limitation Accelerated Graphics Port (AGP), Card Bus, (Extended) Industry Standard Architecture ((E)ISA), Micro Channel Architecture (MCA), NuBus, Peripheral Component Interconnect (Extended) (PCI(X)), PCI Express, Personal Computer Memory Card International Association (PCMCIA), and the like.

The computing architecture 1100 may comprise or implement various articles of manufacture. An article of manufacture may comprise a computer-readable storage medium to store logic. Examples of a computer-readable storage medium may include any tangible media capable of storing electronic data, including volatile memory or non-volatile memory, removable or non-removable memory, erasable or non-erasable memory, writeable or re-writeable memory, and so forth. Examples of logic may include executable computer program instructions implemented using any suitable type of code, such as source code, compiled code, interpreted code, executable code, static code, dynamic code, object-oriented code, visual code, and the like. Embodiments may also be at least partly implemented as instructions contained in or on a non-transitory computer-readable medium, which may be read and executed by one or more processors to enable performance of the operations described herein.

The system memory 1106 may include various types of computer-readable storage media in the form of one or more higher speed memory units, such as read-only memory (ROM), random-access memory (RAM), dynamic RAM (DRAM), Double-Data-Rate DRAM (DDRAM), synchronous DRAM (SDRAM), static RAM (SRAM), programmable ROM (PROM), erasable programmable ROM (EPROM), electrically erasable programmable ROM (EEPROM), flash memory, polymer memory such as ferroelectric polymer memory, ovonic memory, phase change or ferroelectric memory, silicon-oxide-nitride-oxide-silicon (SONOS) memory, magnetic or optical cards, an array of devices such as Redundant Array of Independent Disks (RAID) drives, solid state memory devices (e.g., USB memory, solid state drives (SSD) and any other type of storage media suitable for storing information. In the illustrated embodiment shown in FIG. 11, the system memory 1106 can include non-volatile memory 1110 and/or volatile memory 1112. A basic input/output system (BIOS) can be stored in the non-volatile memory 1110.

The computer 1102 may include various types of computer-readable storage media in the form of one or more lower speed memory units, including an internal (or external) hard disk drive (HDD) 1114A and 1114B, respectively, a magnetic floppy disk drive (FDD) 1116 to read from or write to a removable magnetic disk 1118, and an optical disk drive 1110 to read from or write to a removable optical disk 1122 (e.g., a CD-ROM or DVD). The HDD 1114, FDD 1116 and optical disk drive 1110 can be connected to the system bus 1108 by a HDD interface 1124, an FDD interface 1126 and an optical drive interface 1128, respectively. The HDD interface 1124 for external drive implementations can include at least one or both of Universal Serial Bus (USB) and IEEE 1194 interface technologies.

The drives and associated computer-readable storage media provide volatile and/or nonvolatile storage of data, data structures, computer-executable instructions, and so forth. For example, a number of program components can be stored in the drives and memory units 1110, 1112, including an operating system 1130, one or more application programs 1132, other program components 1134, and program data 1136. In one embodiment, the one or more application programs 1132, other program components 1134, and program data 1136 can include, for example, the various applications and/or components of the system 110.

An operator can enter commands and information into the computer 1102 through one or more wire/wireless input devices, for example, a keyboard 1138 and a pointing device, such as a mouse 1140. Other input devices may include microphones, infra-red (IR) remote controls, radio-frequency (RF) remote controls, game pads, stylus pens, card readers, dongles, finger print readers, gloves, graphics tablets, joysticks, keyboards, retina readers, touch screens (e.g., capacitive, resistive, etc.), trackballs, trackpads, sensors, styluses, and the like. These and other input devices are often connected to the processor circuit 1104 through an input device interface 1142 that is coupled to the system bus 1108, but can be connected by other interfaces such as a parallel port, IEEE 1394 serial port, a game port, a USB port, an IR interface, and so forth.

A monitor 1144 or other type of display device is also connected to the system bus 1108 via an interface, such as a video adaptor 1146. The monitor 1144 may be internal or external to the computer 1102. In addition to the monitor 1144, a computer typically includes other peripheral output devices, such as speakers, printers, and so forth.

The computer 1102 may operate in a networked environment using logical connections via wire and/or wireless communications to one or more remote computers, such as a remote computer 1148. The remote computer 1148 can be a workstation, a server computer, a router, a personal computer, portable computer, microprocessor-based entertainment appliance, a peer device or other common network node, and typically includes many or all of the elements described relative to the computer 1102, although, for purposes of brevity, only a memory/storage device 1150 is illustrated. The logical connections depicted include wire/wireless connectivity to a local area network (LAN) 1152 and/or larger networks, for example, a wide area network (WAN) 1154. Such LAN and WAN networking environments are commonplace in offices and companies, and facilitate enterprise-wide computer networks, such as intranets, all of which may connect to a global communications network, for example, the Internet.

When used in a LAN networking environment, the computer 1102 is connected to the LAN 1152 through a wire and/or wireless communication network interface or adaptor 1156. The adaptor 1156 can facilitate wire and/or wireless communications to the LAN 1152, which may also include a wireless access point disposed thereon for communicating with the wireless functionality of the adaptor 1156.

When used in a WAN networking environment, the computer 1102 can include a modem 1158, or is connected to a communications server on the WAN 1154, or has other means for establishing communications over the WAN 1154, such as by way of the Internet. The modem 1158, which can be internal or external and a wire and/or wireless device, connects to the system bus 1108 via the input device interface 1142. In a networked environment, program components depicted relative to the computer 1102, or portions thereof, can be stored in the remote memory/storage device 1150. It will be appreciated that the network connections shown are exemplary and other means of establishing a communications link between the computers can be used.

The computer 1102 is operable to communicate with wire and wireless devices or entities using the IEEE 802 family of standards, such as wireless devices operatively disposed in wireless communication (e.g., IEEE 802.11 over-the-air modulation techniques). This includes at least Wi-Fi (or Wireless Fidelity), WiMax, and Bluetooth™ wireless technologies, among others. Thus, the communication can be a predefined structure as with a conventional network or simply an ad hoc communication between at least two devices. Wi-Fi networks use radio technologies called IEEE 802.11x (a, b, g, n, etc.) to provide secure, reliable, fast wireless connectivity. A Wi-Fi network can be used to connect computers to each other, to the Internet, and to wire networks (which use IEEE 802.3-related media and functions).

**F**IG. **1**2 illustrates a block diagram of an exemplary communications architecture 1 200 suitable for implementing various embodiments as previously described. The communications architecture 1200 includes various common communications elements, such as a transmitter, receiver, transceiver, radio, network interface, baseband processor, antenna, amplifiers, filters, power supplies, and so forth. The embodiments, however, are not limited to implementation by the communications architecture 1200.

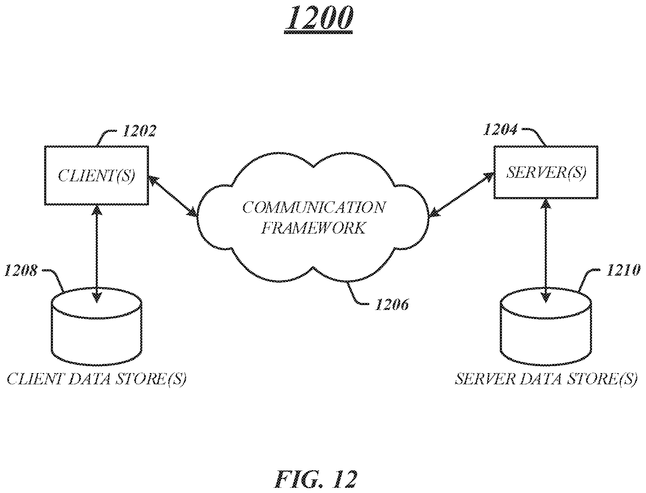

As shown in FIG. 12, the communications architecture 1200 comprises includes one or more clients 1202 and servers 1204. The clients 1202 may implement the devices 910, 920, 950. The servers 1204 may implement any of server devices 110, 210, 1010, 1050. The clients 1202 and the servers 1204 are operatively connected to one or more respective client data stores 1208 and server data stores 1210 that can be employed to store information local to the respective clients 1202 and servers 1204, such as cookies and/or associated contextual information.

The clients 1202 and the servers 1204 may communicate information between each other using a communication framework 1206. The communications framework 1206 may implement any well-known communications techniques and protocols. The communications framework 1206 may be implemented as a packet-switched network (e.g., public networks such as the Internet, private networks such as an enterprise intranet, and so forth), a circuit-switched network (e.g., the public switched telephone network), or a combination of a packet-switched network and a circuit-switched network (with suitable gateways and translators).

The communications framework 1206 may implement various network interfaces arranged to accept, communicate, and connect to a communications network. A network interface may be regarded as a specialized form of an input output interface. Network interfaces may employ connection protocols including without limitation direct connect, Ethernet (e.g., thick, thin, twisted pair 10/100/1000 Base T, and the like), token ring, wireless network interfaces, cellular network interfaces, IEEE 802.11a-x network interfaces, IEEE 802.16 network interfaces, IEEE 802.20 network interfaces, and the like. Further, multiple network interfaces may be used to engage with various communications network types. For example, multiple network interfaces may be employed to allow for the communication over broadcast, multicast, and unicast networks. Should processing requirements dictate a greater amount speed and capacity, distributed network controller architectures may similarly be employed to pool, load balance, and otherwise increase the communicative bandwidth required by clients 1202 and the servers 1204. A communications network may be any one and the combination of wired and/or wireless networks including without limitation a direct interconnection, a secured custom connection, a private network (e.g., an enterprise intranet), a public network (e.g., the Internet), a Personal Area Network (PAN), a Local Area Network (LAN), a Metropolitan Area Network (MAN), an Operating Missions as Nodes on the Internet (OMNI), a Wide Area Network (WAN), a wireless network, a cellular network, and other communications networks.

A machine-readable storage medium may comprise instructions that when executed by a computing device, cause the computing device to generate a medication schedule for a patient, the medication schedule having time slots, each time slot allocated to a different medication event, where a medication event comprises a medication and a dosage. The instructions may cause the device to determine that a first medication event in the medication schedule has an unspecified time requirement comprising a frequency of the medication event without a specific time of day. The instructions may cause the device to determine whether the first medication event has a conflict with a second medication event provided in a second time slot; and move the first medication event to the second time slot in the medication schedule when there is no conflict between the first and second medication events.

The instructions may cause the device to determine whether the medication of a medication event is associated with a chronotherapeutic consideration comprising a preferred time of day for administration; and allocate the medication event to a time slot corresponding to the preferred time of day.

The instructions may cause the device to receive prescription information for the patient, the prescription information comprising, for all non-expired prescriptions, all medications and medication instructions prescribed to the patient at a time when the prescription information is received; and generate the medication schedule according to the medication instructions.

The instructions may cause the device to determine, for each time slot in the medication schedule, whether a first medication event in the time slot conflicts with a second medication in the time slot; and move one of the first and second medication events to a different time slot when there is a conflict between the first and second medication events.

The instructions may cause the device to output the medication schedule to the patient, a pharmacy, or a prescriber. Outputting the medication schedule may comprise printing a label for a medication container for a prescription for the patient, the label comprising the time slots and medication events from the medication schedule that are specific to the prescription.

Some embodiments may be described using the expression "one embodiment" or "an embodiment" along with their derivatives. These terms mean that a particular feature, structure, or characteristic described in connection with the embodiment is included in at least one embodiment. The appearances of the phrase "in one embodiment" in various places in the specification are not necessarily all referring to the same embodiment. Further, some embodiments may be described using the expression "coupled" and "connected" along with their derivatives. These terms are not necessarily intended as synonyms for each other. For example, some embodiments may be described using the terms "connected" and/or "coupled" to indicate that two or more elements are in direct physical or electrical contact with each other. The term "coupled," however, may also mean that two or more elements are not in direct contact with each other, but yet still co-operate or interact with each other.

It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, it can be seen that various features are grouped together in a single embodiment for the purpose of streamlining the disclosure. This method of disclosure is not to be interpreted as reflecting an intention that the claimed embodiments require more features than are expressly recited in each claim. Rather, as the following claims reflect, inventive subject matter lies in less than all features of a single disclosed embodiment. Thus the following claims are hereby incorporated into the Detailed Description, with each claim standing on its own as a separate embodiment. In the appended claims, the terms "including" and "in which" are used as the plain-English equivalents of the respective terms "comprising" and "wherein," respectively. Moreover, the terms "first," "second," "third," and so forth, are used merely as labels, and are not intended to impose numerical requirements on their objects.

What has been described above includes examples of the disclosed architecture. It is, of course, not possible to describe every conceivable combination of components and/or methodologies, but one of ordinary skill in the art may recognize that many further combinations and permutations are possible. Accordingly, the novel architecture is intended to embrace all such alterations, modifications and variations that fall within the scope of the appended claims.

As used herein, an element or step recited in the singular and proceed with the word "a" or "an" should be understood as not excluding plural elements or steps, unless such exclusion is explicitly recited. Furthermore, references to "one embodiment" of the present invention are not intended to be interpreted as excluding the existence of additional embodiments that also incorporate the recited features.

While certain embodiments of the disclosure have been described herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A method implemented by an automatic medication scheduling system, the method comprising:
generating, at an automatic medication scheduling server of the automatic medication scheduling system, an initial medication schedule for a patient, wherein the medication schedule comprises a plurality of days each divided into at least two time slots and wherein times slot are allocated to at least one medication event comprising taking a medication at a dosage;
querying, at the automatic medication scheduling server, at least one drug database for conflict information associated with the medication events in the initial medication schedule;
at the automatic medication scheduling server, iteratively, for each medication event in the initial medication schedule, determining, by analyzing the conflict information, whether the medication event conflicts with another medication event in the initial medication schedule and, if so, reassigning one of the medication event and the other medication event to a time slot that is different to that to which the one of the medication event and the other medication is currently assigned, wherein the iterative determining is repeated until all conflicts have been resolved or, in the event that all conflicts cannot be resolved, a predetermined number of repetitions has been performed, wherein the time slot to which the one of the medication event and the other medication is reassigned is a time slot which resolves the determined conflict;
subsequently, examining, at the automatic medication scheduling server, time slots which have only one medication event assigned to the time slot;
determining, at the automatic medication scheduling server, that a first medication event, which is the only medication event assigned to a particular time slot, has an unspecified time-of-day taking requirement;
at the automatic medication scheduling server and responsive to determining that the first medication event, which is the only medication event assigned to a particular time slot, has an unspecified time-of-day taking requirement, identifying a candidate time slot to which a second medication event is assigned, wherein the candidate time slot is different to the particular time slot;
at the automatic medication scheduling server, analyzing the conflict information to determine whether the first medication event conflicts with the second medication event assigned to the candidate time slot;
re-assigning, at the automatic medication scheduling server, the first medication event to the candidate time slot in the medication schedule when it is determined that there is no conflict between the first and second medication events; and
electronically outputting from the medication scheduling server the medication schedule to the patient, a pharmacy, or a prescriber.

2. The method of claim 1, further comprising:
generating the initial medication schedule by:
receiving prescription information for the patient, the prescription information comprising all medications and medication instructions prescribed to the patient at a time when the prescription information is received; and
assigning the medication events to the time slots in the initial schedule according to the medication instructions.

3. The method of claim 2, further comprising:
using natural language processing or keyword matching to interpret the medication instructions to identify at least one of a time of dosage and a frequency of dosage.

4. The method of claim 2, wherein generating the initial medication schedule comprises:
determining whether any of the medications of the medication events of the initial medication schedule are associated with chronotherapeutic considerations comprising a preferred time of day for administration or a non-preferred time of day for administration;
if so, determining whether the time slot to which the medication associated with a chronotherapeutic consideration is assigned corresponds to the preferred or non-preferred time of day; and
moving the medication event associated with the chronotherapeutic consideration to a time slot corresponding to the preferred time of day when the allocated time slot does not correspond to the preferred time of day or from a time slot corresponding to the non-preferred time of day when the allocated time slot does correspond to the non-preferred time of day.

5. The method of claim 1, further comprising:
presenting the medication schedule to the patient.

6. An automatic medication scheduling server comprising:
one or more processor circuits;
a scheduling component that, when executing on a processor circuit, generates an initial medication schedule for a patient, the medication schedule having at least two time slots, each time slot allocated to a different medication event, a medication event comprising a medication and a dosage;
a drug interaction component that, when executing on a processor circuit, determines, iteratively for each medication event in the initial medication schedule and by analyzing the conflict information, whether the medication event conflicts with another medication event in the initial medication schedule and, if so, reassigning one of the medication event and the other medication event to a time slot that is different to that to which the one of the medication event and the other medication is currently assigned, wherein the iterative determining is repeated until all conflicts have been resolved or, in the event that all conflicts cannot be resolved, a predetermined number of repetitions has been performed, wherein the time slot to which the one of the medication event and the other medication is reassigned is a time slot which resolves the determined conflict; and
a consolidation component that, when executing on a processor circuit, subsequently:
examines time slots which have only one medication event assigned to the time slot,
determines that a first medication event, which is the only medication event assigned to a particular time slot, has an unspecified time-of-day taking requirement,
identifies, responsive to determining that the first medication event has an unspecified time-of-day taking requirement, a candidate time slot to which a second medication event is assigned, wherein the candidate time slot is different to the particular time slot,
analyzes the conflict information to determine whether the first medication event conflicts with the second medication event assigned to the candidate time slot, and
reassigns the first medication event from the particular time slot to the candidate time slot when it is determined that there is no conflict between the first medication event and the second medication event, thereby to reduce the total number of time slots in the medication schedule that have an assigned medication event,
wherein the scheduling component outputs the medication schedule to the patient, a pharmacy, or a prescriber.

7. The apparatus of claim 6, wherein the scheduling component receives prescription information for the patient, the prescription information comprising all medications and medication instructions prescribed to the patient at the time when the prescription information is received; and wherein the scheduling component generates the initial medication schedule according to the medication instructions.

8. The apparatus of claim 7, wherein the scheduling component receives the prescription information from one or more of a retail pharmacy, a specialty pharmacy, or a mail order pharmacy, and receives information about chronotherapeutic considerations and drug side effects from one or more of pharmacy database, a commercial drug database, a proprietary drug database, and a governmental drug database.

9. The apparatus of claim 6, wherein the drug interaction component determines a conflict comprising at least one of: an adverse drug interaction when two or more medications are taken within a first time threshold of each other, and a decreased effectiveness interaction when two or more medications are taken within a second time threshold of each other.

10. The apparatus of claim 6, wherein the apparatus is configured cause a label for a medication container for a prescription for the patient to be printed, the label comprising the time slots and medication events from the medication schedule that are specific to the prescription.

11. At least one machine-readable storage medium comprising instructions that when executed by a computing device, cause the computing device to:
generate, at an automatic medication scheduling server of a automatic medication scheduling system, an initial medication schedule for a patient, wherein the medication schedule comprises a plurality of days each divided into at least two time slots and wherein times slot are allocated to at least one medication event comprising taking a medication at a dosage;
query, at the automatic medication scheduling server, at least one drug database for conflict information associated with the medication events in the initial medication schedule
at the automatic medication scheduling server, iteratively, for each medication event in the initial medication schedule, determine, by analyzing the conflict information, whether the medication event conflicts with another medication event in the initial medication schedule and, if so, reassigning one of the medication event and the other medication event to a time slot that is different to that to which the one of the medication event and the other medication is currently assigned, wherein the iterative determining is repeated until all conflicts have been resolved or, in the event that all conflicts cannot be resolved, a predetermined number of repetitions has been performed, wherein the time slot to which the one of the medication event and the other medication is reassigned is a time slot which resolves the determined conflict;
subsequently, examine, at the automatic medication scheduling server, time slots which have only one medication event assigned to the time slot;
determine, at the automatic medication scheduling server, that a first medication event, which is the only medication event assigned to a particular time slot, has an unspecified time-of-day taking requirement;
responsive to determining that the first medication event, which is the only medication event assigned to a particular time slot, has an unspecified time-of-day taking requirement, identify a candidate time slot to which a second medication event is assigned, wherein the candidate time slot is different to the particular time slot;
at the automatic medication scheduling server, analyze the conflict information to determine whether the first medication event conflicts with the second medication event assigned to the candidate time slot;
re-assign, at the automatic medication scheduling server, the first medication event to the candidate time slot in the medication schedule when it is determined that there is no conflict between the first and second medication events; and
electronically output from the medication scheduling server the medication schedule to the patient, a pharmacy, or a prescriber.

## Patentansprüche

1. Verfahren, implementiert durch ein automatisches Medikamentenplanungssystem, wobei das Verfahren Folgendes umfasst:
Erzeugen, an einem automatischen Medikamentenplanungsserver des automatischen Medikamentenplanungssystems, eines anfänglichen Medikamentenplans für einen Patienten, wobei der Medikamentenplan eine Vielzahl von Tagen umfasst, die jeweils in mindestens zwei Zeitfenster unterteilt sind, und wobei die Zeitfenster zumindest einem Medikamentenereignis zugewiesen sind, das die Einnahme eines Medikaments in einer bestimmten Dosierung umfasst;
Abfragen, an dem automatischen Medikamentenplanungsserver, zumindest einer Medikamentendatenbank für Konfliktinformationen, die mit den Medikamentenereignissen in der anfänglichen Medikamentenplanung assoziiert sind;
an dem automatischen Medikamentenplanungsserver iterativ für jedes Medikamentenereignis in der anfänglichen Medikamentenplanung, Ermitteln, durch Analysieren der Konfliktinformationen, ob das Medikamentenereignis mit einem anderen Medikamentenereignis in der anfänglichen Medikamentenplanung kollidiert, und, falls dies der Fall ist, Neuzuordnen eines des Medikamentenereignisses und des anderen Medikamentenereignisses zu einem Zeitfenster, das sich von demjenigen unterscheidet, dem das eine des Medikamentenereignisses und des anderen Medikaments zurzeit zugewiesen ist, wobei das iterative Ermitteln wiederholt wird, bis alle Konflikte gelöst wurden oder, für den Fall, dass alle Konflikte nicht gelöst werden können, eine vorbestimmte Anzahl an Wiederholungen durchgeführt wurde, wobei das Zeitfenster, dem das eine von dem Medikamentenereignis und dem anderen Medikament neu zugewiesen wird, ein Zeitfenster ist, das den ermittelten Konflikt löst;
anschließendes Untersuchen, an dem automatischen Medikamentenplanungsserver, der Zeitfenster, die nur ein Medikamentenereignis aufweisen, das dem Zeitfenster zugewiesen ist;
Ermitteln, an dem automatischen Medikamentenplanungsserver, dass ein erstes Medikamentenereignis, welches das einzige einem bestimmten Zeitfenster zugewiesene Medikamentenereignis ist, eine nicht spezifizierte Tageszeit für die Einnahme aufweist;
an dem automatischen Medikamentenplanungsserver und in Reaktion auf das Ermitteln, dass das erste Medikamentenereignis, welches das einzige einem bestimmten Zeitfenster zugewiesene Medikamentenereignis ist, eine nicht spezifizierte Tageszeit für die Einnahme aufweist, Identifizieren eines Kandidatenzeitfensters, dem ein zweites Medikamentenereignis zugewiesen ist, wobei das Kandidatenzeitfenster von dem bestimmten Zeitfenster verschieden ist;
an dem automatischen Medikamentenplanungsserver, Analysieren der Konfliktinformationen, um zu ermitteln, ob das erste Medikamentenereignis mit dem zweiten Medikamentenereignis, das dem Zeitfenster des Kandidaten zugewiesen ist, in Konflikt steht;
erneutes Zuweisen, an dem automatischen Medikamentenplanungsserver, des ersten Medikamentenereignisses zu dem Kandidatenzeitfenster in der Medikamentenplanung, wenn ermittelt wird, dass es keinen Konflikt zwischen dem ersten und dem zweiten Medikamentenereignis gibt; und
elektronisches Ausgeben von dem Medikamentenplanungsserver der Planung der Medikation für den Patienten, eine Apotheke oder einen verschreibenden Arzt.

2. Verfahren nach Anspruch 1, ferner umfassend:
Erzeugen der anfänglichen Medikamentenplanung durch:
Empfangen von Rezeptinformationen für den Patienten, wobei die Rezeptinformationen alle Medikamente und Medikamentenanweisungen umfassen, die dem Patienten zu einem Zeitpunkt verschrieben werden, wenn die Rezeptinformationen empfangen werden; und
Zuweisen der Medikamentenereignisse zu den Zeitfenstern in der anfänglichen Planung gemäß den Medikamentenanweisungen.

3. Verfahren nach Anspruch 2, ferner umfassend:
Verwendung einer Verarbeitung natürlicher Sprache oder eines Schlüsselwortabgleichs zur Interpretation der Anweisungen für die Medikation, um zumindest einen Zeitpunkt der Dosierung oder eine Häufigkeit der Dosierung zu identifizieren.

4. Verfahren nach Anspruch 2, wobei das Erzeugen der anfänglichen Medikamentenplanung Folgendes umfasst:
Ermitteln, ob irgendeines der Medikamente der Medikamentenereignisse der anfänglichen Medikamentenplanung mit chronotherapeutischen Überlegungen assoziiert ist, die einen bevorzugten Tageszeitpunkt für die Verabreichung oder einen nicht bevorzugten Tageszeitpunkt für die Verabreichung umfassen;
wenn ja, Ermitteln, ob der Zeitpunkt, dem das mit einer chronotherapeutischen Überlegung assoziierte Medikament zugewiesen ist, der bevorzugten oder nicht bevorzugten Tageszeit entspricht; und
Verschieben des mit der chronotherapeutischen Überlegung assoziierten Medikamentenereignisses zu einem Zeitfenster, das dem bevorzugten Tageszeitpunkt entspricht, wenn das zugewiesene Zeitfenster nicht dem bevorzugten Tageszeitpunkt entspricht, oder von einem Zeitfenster, das dem nicht bevorzugten Tageszeitpunkt entspricht, wenn das zugewiesene Zeitfenster dem nicht bevorzugten Tageszeitpunkt entspricht.

5. Verfahren nach Anspruch 1, ferner umfassend:
Präsentieren der Medikamentenplanung dem Patienten.

6. Automatischer Medikamentenplanungsserver, umfassend:
eine oder mehrere Prozessorschaltungen;
eine Planungskomponente, die bei Ausführung auf einer Prozessorschaltung eine anfängliche Medikamentenplanung für einen Patienten erzeugt, wobei die Medikamentenplanung zumindest zwei Zeitfenster aufweist, wobei jedes Zeitfenster einem anderen Medikamentenereignis zugeordnet ist, wobei ein Medikamentenereignis ein Medikament und eine Dosierung umfasst;
eine Komponente für Wechselwirkungen von Medikamenten, die bei Ausführung durch eine Prozessorschaltung iterativ für jedes Ereignis in der anfänglichen Medikamentenplanung und durch Analysieren der Konfliktinformationen ermittelt, ob das Medikamentenereignis mit einem anderen Medikamentenereignis in der anfänglichen Medikamentenplanung kollidiert, und, falls dies der Fall ist, Neuzuordnen eines des Medikamentenereignisses und des anderen Medikamentenereignisses zu einem Zeitfenster, das sich von demjenigen unterscheidet, dem das eine des Medikamentenereignisses und des anderen Medikaments zurzeit zugewiesen ist, wobei das iterative Ermitteln wiederholt wird, bis alle Konflikte gelöst wurden oder, für den Fall, dass alle Konflikte nicht gelöst werden können, eine vorbestimmte Anzahl an Wiederholungen durchgeführt wurde, wobei das Zeitfenster, dem das eine von dem Medikamentenereignis und dem anderen Medikament neu zugewiesen wird, ein Zeitfenster ist, das den ermittelten Konflikt löst; und eine Konsolidierungskomponente, die bei Ausführung auf einer Prozessorschaltung, anschließend:
Zeitfenster untersucht, die nur ein dem Zeitfenster zugewiesenes Medikamentenereignis aufweisen,
Ermittelt, dass ein erstes Medikamentenereignis, welches das einzige einem bestimmten Zeitfenster zugewiesene Medikamentenereignis ist, eine nicht spezifizierte Tageszeit für die Einnahme aufweist,
Identifizieren, in Reaktion auf das Ermitteln, dass das erste Medikamentenereignis eine nicht spezifizierte Tageszeit für die Einnahme aufweist, eines Kandidatenzeitfensters, dem ein zweites Medikamentenereignis zugewiesen ist, wobei das Kandidatenzeitfenster von dem bestimmten Zeitfenster verschieden ist,
Analysieren der Konfliktinformationen, um zu ermitteln, ob das erste Medikamentenereignis mit dem zweiten Medikamentenereignis, das dem Zeitfenster des Kandidaten zugewiesen ist, in Konflikt steht, und
erneutes Zuweisen des ersten Medikamentenereignisses aus dem bestimmten Zeitfenster in das Kandidatenzeitfenster, wenn ermittelt wird, dass kein Konflikt zwischen dem ersten Medikamentenereignis und dem zweiten Medikamentenereignis besteht, um so die Gesamtanzahl der Zeitfenster in der Medikamentenplanung zu reduzieren, die ein zugewiesenes Medikamentenereignis aufweisen,
wobei die Planungskomponente die Medikamentenplanung an den Patienten, eine Apotheke oder einen verschreibenden Arzt ausgibt.

7. Vorrichtung nach Anspruch 6, wobei die Planungskomponente Rezeptinformationen für den Patienten empfängt, wobei die Rezeptinformationen alle Medikamente und Medikamentenanweisungen umfassen, die dem Patienten zu dem Zeitpunkt, zu dem die Rezeptinformationen empfangen werden, verordnet werden; und wobei die Planungskomponente die anfängliche Medikamentenplanung gemäß den Medikamentenanweisungen erzeugt.

8. Vorrichtung nach Anspruch 7, wobei die Planungskomponente die Rezeptinformationen von einer oder mehreren Einzelhandelsapotheken, Spezialapotheken oder Versandapotheken empfängt und Informationen über chronotherapeutische Betrachtungen und Arzneimittelnebenwirkungen von einer oder mehreren Apothekendatenbanken, einer kommerziellen Arzneimitteldatenbank, einer proprietären Arzneimitteldatenbank und einer staatlichen Arzneimitteldatenbank empfängt.

9. Vorrichtung nach Anspruch 6, wobei die Komponente für Wechselwirkungen von Medikamenten einen Konflikt ermittelt, der zumindest eines von Folgendem umfasst: unerwünschte Wechselwirkung von Medikamenten, wenn zwei oder mehr Medikamente innerhalb eines ersten Zeitpunkts voneinander eingenommen werden, und Wechselwirkung mit verminderter Wirksamkeit, wenn zwei oder mehr Medikamente innerhalb eines zweiten Zeitpunkts voneinander eingenommen werden.

10. Vorrichtung nach Anspruch 6, wobei die Vorrichtung konfiguriert ist, zu veranlassen, dass ein Etikett für einen Medikamentenbehälter für ein Rezept für den Patienten gedruckt wird, wobei das Etikett die Zeitfenster und Medikamentenereignisse aus der Medikamentenplanung umfasst, die für das Rezept spezifisch sind.

11. Zumindest ein maschinenlesbares Speichermedium, das Anweisungen umfasst, die bei Ausführung durch ein Computergerät, das Computergerät zu folgendem veranlassen:
Erzeugen, an einem automatischen Medikamentenplanungsserver eines automatischen Medikamentenplanungssystems, eines anfänglichen Medikamentenplans für einen Patienten, wobei der Medikamentenplan eine Vielzahl von Tagen umfasst, die jeweils in mindestens zwei Zeitfenster unterteilt sind, und wobei die Zeitfenster zumindest einem Medikamentenereignis zugewiesen sind, das die Einnahme eines Medikaments in einer bestimmten Dosierung umfasst;
Abfragen, an dem automatischen Medikamentenplanungsserver, zumindest einer Arzneimitteldatenbank für Konfliktinformationen, die mit den Medikamentenereignissen in der anfänglichen Medikamentenplanung assoziiert sind, an dem automatischen Medikamentenplanungsserver, iteratives Ermitteln für jedes Medikamentenereignis in der anfänglichen Medikamentenplanung, durch Analysieren der Konfliktinformationen, ob das Medikamentenereignis mit einem anderen Medikamentenereignis in der anfänglichen Medikamentenplanung kollidiert, und, falls dies der Fall ist, Neuzuordnen eines des Medikamentenereignisses und des anderen Medikamentenereignisses zu einem Zeitfenster, das sich von demjenigen unterscheidet, dem das eine des Medikamentenereignisses und des anderen Medikaments zurzeit zugewiesen ist, wobei das iterative Ermitteln wiederholt wird, bis alle Konflikte gelöst wurden oder, für den Fall, dass alle Konflikte nicht gelöst werden können, eine vorbestimmte Anzahl an Wiederholungen durchgeführt wurde, wobei das Zeitfenster, dem das eine von dem Medikamentenereignis und dem anderen Medikament neu zugewiesen wird, ein Zeitfenster ist, das den ermittelten Konflikt löst;
anschließendes Untersuchen, an dem automatischen Medikamentenplanungsserver, der Zeitfenster, die nur ein Medikamentenereignis aufweisen, das dem Zeitfenster zugewiesen ist;
Ermitteln, an dem automatischen Medikamentenplanungsserver, dass ein erstes Medikamentenereignis, welches das einzige einem bestimmten Zeitfenster zugewiesene Medikamentenereignis ist, eine nicht spezifizierte Tageszeit für die Einnahme aufweist;
in Reaktion auf das Ermitteln, dass das erste Medikamentenereignis, welches das einzige einem bestimmten Zeitfenster zugewiesene Medikamentenereignis ist, eine nicht spezifizierte Tageszeit für die Einnahme aufweist, Identifizieren eines Kandidatenzeitfensters, dem ein zweites Medikamentenereignis zugewiesen ist, wobei das Kandidatenzeitfenster von dem bestimmten Zeitfenster verschieden ist;
an dem automatischen Medikamentenplanungsserver, Analysieren der Konfliktinformationen, um zu ermitteln, ob das erste Medikamentenereignis mit dem zweiten Medikamentenereignis, das dem Zeitfenster des Kandidaten zugewiesen ist, in Konflikt steht;
erneutes Zuweisen, an dem automatischen Medikamentenplanungsserver, des ersten Medikamentenereignisses zu dem Kandidatenzeitfenster in der Medikamentenplanung, wenn ermittelt wird, dass es keinen Konflikt zwischen dem ersten und dem zweiten Medikamentenereignis gibt; und
elektronisches Ausgeben von dem Medikamentenplanungsserver der Planung der Medikation für den Patienten, eine Apotheke oder einen verschreibenden Arzt.

## Revendications

1. Procédé mis en œuvre par un système de programmation automatique de médication, le procédé comprenant :
la génération, au niveau d'un serveur de programmation automatique de médication du système de programmation automatique de médication, d'un programme de médication initial pour un patient, dans lequel le programme de médication comprend une pluralité de jours, chacun divisé en au moins deux intervalles de temps et dans lequel des intervalles de temps sont attribués à au moins un événement de médication comprenant la prise d'une médication à un dosage ;
l'interrogation, au niveau du serveur de programmation automatique de médication, d'au moins une base de données de médicaments pour des informations contradictoires associées aux événements de médications dans le programme de médication initial ;
au niveau du serveur de programmation automatique de médication, de manière itérative, pour chaque événement de médication dans le programme de médication initial, le fait de déterminer, en analysant les informations contradictoires, si l'événement de médication est en contradiction avec un autre événement de médication dans le programme de médication initial et, si oui, la réattribution de l'un parmi l'événement de médication et l'autre événement de médication à un intervalle de temps qui est différent de celui auquel l'un parmi l'événement de médication et l'autre médication est actuellement attribué, dans lequel la détermination itérative est répétée jusqu'à ce que toutes les contradictions aient été résolues ou, dans le cas où toutes les contradictions ne peuvent pas être résolues, un nombre prédéterminé de répétitions a été réalisé, dans lequel l'intervalle de temps auquel l'un parmi l'événement de médication ou l'autre médication est réattribué est un intervalle de temps qui résout la contradiction déterminée ;
par la suite, l'examen, au niveau du serveur de programmation automatique de médication, d'intervalles de temps qui n'ont qu'un événement de médication attribué à l'intervalle de temps ;
la détermination, au niveau du serveur de programmation automatique de médication, qu'un premier événement de médication, qui est le seul événement de médication attribué à un intervalle de temps particulier, a une exigence d'heure de prise non spécifiée ;
au niveau du serveur de programmation automatique de médication et en réponse à la détermination que le premier événement de médication, qui est le seul événement de médication attribué à un intervalle de temps particulier, a une exigence d'heure de prise non spécifiée, l'identification d'un intervalle de temps candidat auquel un second événement de médication est attribué, dans lequel l'intervalle de temps candidat est différent de l'intervalle de temps particulier ;
au niveau du serveur de programmation automatique de médication, l'analyse des informations contradictoires pour déterminer si le premier événement de médication est contradictoire avec le second événement de médication attribué à l'intervalle de temps candidat ;
la réattribution, au niveau du serveur de programmation automatique de médication, du premier événement de médication à l'intervalle de temps candidat dans le programme de médication lorsqu'il est déterminé qu'il n'y a aucune contradiction entre les premier et second événements de médication ; et
la délivrance par voie électronique au patient, à une pharmacie ou à un prescripteur, du programme de médication à partir du serveur de programmation de médication.

2. Procédé selon la revendication 1, comprenant en outre :
la génération du programme de médication initial par :
la réception d'informations de prescription pour le patient, les informations de prescription comprenant toutes les médications et instructions de médications prescrites au patient à un temps auquel les informations de prescription sont reçues ; et
l'attribution des événements de médication aux intervalles de temps dans le programme initial conformément aux instructions de médication.

3. Procédé selon la revendication 2, comprenant en outre :
l'utilisation d'un traitement en langage naturel ou d'une mise en correspondance de mots clés pour interpréter les instructions de médication afin d'identifier au moins un temps de dosage et une fréquence de dosage.

4. Procédé selon la revendication 2, dans lequel la génération du programme de médication initial comprend :
le fait de déterminer si l'une quelconque des médications des événements de médication du programme de médication initial est associée à des considérations chronothérapeutiques comprenant une heure du jour préférée pour l'administration ou une heure du jour non préférée pour l'administration ;
si oui, le fait de déterminer si l'intervalle de temps auquel la médication associée à une considération chronothérapeutique est attribuée correspond à l'heure du jour préférée ou non préférée ; et
le déplacement de l'événement de médication associé à la considération chronothérapeutique jusqu'à un intervalle de temps correspondant à l'heure du jour préférée lorsque l'intervalle de temps attribué ne correspond pas à l'heure du jour préférée ou à partir d'un intervalle de temps correspondant à l'heure du jour non préférée lorsque l'intervalle de temps attribué correspond à l'heure du jour non préférée.

5. Procédé selon la revendication 1, comprenant en outre :
la présentation du programme de médication au patient.

6. Serveur de programmation automatique de médication comprenant :
un ou plusieurs circuits de processeur ;
un composant de programmation qui, lorsqu'il s'exécute sur un circuit de processeur, génère un programme de médication initial pour un patient, le programme de médication ayant au moins deux intervalles de temps, chaque intervalle de temps étant attribué à un événement de médication différent, un événement de médication comprenant une médication et un dosage ;
un composant d'interaction médicamenteuse qui, lors de l'exécution sur un circuit de processeur, détermine, de manière itérative pour chaque événement de médication dans le programme de médication initial et par l'analyse des informations contradictoires, si l'événement de médication est en contradiction avec un autre événement de médication dans le programme de médication initial et, si oui, la réattribution de l'un parmi l'événement de médication et l'autre événement de médication à un intervalle de temps qui est différent de celui auquel l'un parmi l'événement de médication et l'autre médication est actuellement attribué, dans lequel la détermination itérative est répétée jusqu'à ce que toutes les contradictions aient été résolues ou, dans le cas où toutes les contradictions ne peuvent pas être résolues, un nombre prédéterminé de répétitions a été réalisé, dans lequel l'intervalle de temps auquel l'un parmi l'événement de médication ou l'autre médication est réattribué est un intervalle de temps qui résout la contradiction déterminée ; et
un composant de consolidation qui, lors de l'exécution sur un circuit de processeur, par la suite :
examine les intervalles de temps qui n'ont qu'un événement de médication attribué à l'intervalle de temps,
détermine qu'un premier événement de médication, qui est le seul événement de médication attribué à un intervalle de temps particulier, a une exigence d'heure de prise non spécifiée,
en réponse à la détermination que le premier événement de médication a une exigence d'heure de prise non spécifiée, identifie un intervalle de temps candidat auquel un second événement de médication est attribué, dans lequel l'intervalle de temps candidat est différent de l'intervalle de temps particulier,
analyse les informations contradictoires pour déterminer si le premier événement de médication est contradictoire avec le second événement de médication attribué à l'intervalle de temps candidat, et
réattribue le premier événement de médication de l'intervalle de temps particulier à l'intervalle de temps candidat lorsqu'il est déterminé qu'il n'y a pas de contradiction entre le premier événement de médication et le second événement de médication, réduisant ainsi le nombre total d'intervalles de temps dans le programme de médication qui ont un événement de médication attribué,
dans lequel le composant de programmation délivre la programmation de médication au patient, à une pharmacie ou à un prescripteur.

7. Appareil selon la revendication 6, dans lequel le composant de programmation reçoit des informations de prescription pour le patient, les informations de prescription comprenant toutes les médications et instructions de médication prescrites au patient au temps auquel les informations de prescription sont reçues ; et dans lequel le composant de programmation génère le programme de médication initial selon les instructions de médication.

8. Appareil selon la revendication 7, dans lequel le composant de programmation reçoit les informations de prescription depuis un ou plusieurs parmi une pharmacie de détail, une pharmacie spécialisée ou une pharmacie d'achat par correspondance, et reçoit des informations concernant des considérations chronothérapeutiques et des effets secondaires de médicaments depuis une ou plusieurs parmi une base de données de pharmacie, une base de données commerciale de médicaments, une base de données de médicaments de marque déposée et une base de données gouvernementale de médicaments.

9. Appareil selon la revendication 6, dans lequel le composant d'interaction médicamenteuse détermine une contradiction comprenant au moins l'une parmi : une interaction médicamenteuse indésirable lorsque deux médicaments ou plus sont pris dans un premier seuil de temps l'un par rapport à l'autre, et une interaction d'efficacité diminuée lorsque deux médicaments ou plus sont pris dans un second seuil de temps l'un par rapport à l'autre.

10. Appareil selon la revendication 6, dans lequel l'appareil est configuré pour amener l'impression d'une étiquette d'un récipient de médication d'une prescription pour le patient, l'étiquette comprenant les intervalles de temps et les événements de médication du programme de médication qui sont spécifiques à la prescription.

11. Au moins un support de stockage lisible par machine comprenant des instructions qui, lorsqu'elles sont exécutées par un dispositif informatique, amènent le dispositif informatique à :
générer, au niveau d'un serveur de programmation automatique de médication du système de programmation automatique de médication, un programme de médication initial pour un patient, dans lequel le programme de médication comprend une pluralité de jours, chacun divisé en au moins deux intervalles de temps et dans lequel des intervalles de temps sont attribués à au moins un événement de médication comprenant la prise d'une médication à un dosage ;
interroger, au niveau du serveur de programmation automatique de médication, au moins une base de données de médications pour des informations contradictoires associées aux événements de médication dans le programme de médication initial au niveau du serveur de programmation automatique de médication, de manière itérative, pour chaque événement de médication dans le programme de médication initial, déterminer, en analysant les informations contradictoires, si l'événement de médication est en contradiction avec un autre événement de médication dans le programme de médication initial et, si oui, la réattribution de l'un parmi l'événement de médication et l'autre événement de médication à un intervalle de temps qui est différent de celui auquel l'un parmi l'événement de médication et l'autre médication est actuellement attribué, dans lequel la détermination itérative est répétée jusqu'à ce que toutes les contradictions aient été résolues ou, dans le cas où toutes les contradictions ne peuvent pas être résolues, un nombre prédéterminé de répétitions a été réalisé, dans lequel l'intervalle de temps auquel l'un parmi l'événement de médication ou l'autre médication est réattribué est un intervalle de temps qui résout la contradiction déterminée ;
par la suite, examiner, au niveau du serveur de programmation automatique de médication, des intervalles de temps qui n'ont qu'un événement de médication attribué à l'intervalle de temps ;
déterminer, au niveau du serveur de programmation automatique de médication, qu'un premier événement de médication, qui est le seul événement de médication attribué à un intervalle de temps particulier, a une exigence d'heure de prise non spécifiée ;
en réponse à la détermination que le premier événement de médication, qui est le seul événement de médication attribué à un intervalle de temps particulier, a une exigence d'heure de prise non spécifiée, identifier un intervalle de temps candidat auquel un second événement de médication est attribué, dans lequel l'intervalle de temps candidat est différent de l'intervalle de temps particulier ;
au niveau du serveur de programmation automatique de médication, analyser les informations contradictoires pour déterminer si le premier événement de médication est contradictoire avec le second événement de médication attribué à l'intervalle de temps candidat ;
réattribuer, au niveau du serveur de programmation automatique de médication, le premier événement de médication à l'intervalle de temps candidat dans le programme de médication lorsqu'il est déterminé qu'il n'y a aucune contradiction entre les premier et second événements de médication ; et
délivrer par voie électronique au patient, à une pharmacie ou à un prescripteur, du programme de médication à partir du serveur de programmation de médication.
